(19) Europäisches Patentamt
European Patent Office
Office européen des brevets

(11) **EP 1 057 455 A2**

(12) **EUROPEAN PATENT APPLICATION**

(43) Date of publication:
06.12.2000 Bulletin 2000/49

(51) Int Cl.⁷: **A61B 19/00**

(21) Application number: **00304690.1**

(22) Date of filing: **02.06.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **04.06.1999 US 326128**
**22.05.2000 US 575451**

(71) Applicant: **ECLIPSE SURGICAL TECHNOLOGIES, Inc.**
**Sunnyvale, California 94089 (US)**

(72) Inventors:
• **Rosinko, Michael J.**
**San Jose, California 95125 (US)**
• **Murphy-Chutorian, Douglas R.**
**Palo Alto, California 94301 (US)**
• **Bartels, Keith A.**
**San Antonio, Texas, 78209 (US)**
• **Roush, Mark**
**Columbia, Illinois 62236 (US)**
• **Kesten, Randy J.**
**Mountain View, California 94043 (US)**

(74) Representative: **Calderbank, Thomas Roger et al**
**MEWBURN ELLIS**
**York House**
**23 Kingsway**
**London WC2B 6HP (GB)**

(54) **Enhanced surgical device tracking system**

(57) A surgical device tracking system for visualization of the position and orientation of a surgical device during a surgical procedure, the system comprising a surgical device comprising at least one sensor, an input instrument, adapted to communicate with one or more sensor on the surgical device, for generating position and orientation data, a processing system for acquiring and interpreting the data and generating a plurality of outputs, and a display system for viewing at least one of the generated outputs. The processing system being capable of receiving and analyzing a descriptive input of the surgical device incorporating the sensor, and determining physical parameters relative to a tissue structure and the relative position, orientation and motion of the surgical device therein, the processing system also acquiring treatment location data.

FIG. 1

**Description**

This invention relates to the field of computer based viewing hardware with fluoroscopic tools for cardiac surgery, and more particularly to viewing devices for myocardial revascularization.

Heart disease is a significant health problem which has been the subject of substantial medical study. Bypass surgery has become commonplace; yet such surgery may be unavailable to many patients, either because of the nature of the occlusions or the physical condition of the patient. One promising alternative technique for treating such cases is known as transmyocardial revascularization (TMR). Although this technique was considered as early as the work of Dr. C. Beck in "The Development of a New Blood Supply to the Heart By Operation", Annals of Surgery, Vol. 102, No. 5 (11/35), pp. 801-813, the method was not extensively studied until the work of Dr. M. Mirhoseini and M. Cayton, an example of which is found in "Lasers in Cardiothoracic Surgery" in Lasers in General Surgery, (Williams and Williams; 1989), pp. 216-223.

Myocardial revascularization systems used by interventional cardiologists include a percutaneous transluminal myocardial revascularization (PMR) instrument that is a catheter and tissue removal energy delivery system that creates channels partially into the myocardium from inside the left ventricle. In the PMR procedure, an interventional cardiologist performs a cardiac catheterization procedure using a catheter with an internal optical fiber which is inserted into the femoral artery at the groin and advanced through the aortic arch into the left ventricle of the heart. Once in the ventricle, the catheter is guided to the endocardium where the device creates pathways through the endocardium and a portion of the myocardium.

A PMR procedure generally requires a physician to use a hand-held device which guides a mechanical cutting device or other suitable energy delivery device. For example, an energy delivery device may include a laser energy device having one or more optical fibers through which laser energy is directed. Mechanical, laser energy, or other suitable energy cuts or vaporizes heart muscle tissue immediately in front of the distal end of the device. Further, varying penetration depths of the energy delivery device within the myocardial tissue are possible. Clinical tests have demonstrated that revascularization channels/pathways, which generally communicate with the ventricle, facilitate revascularization of the heart muscle.

U.S. Patent No. 5,876,373 to Giba et al., entitled "Steerable Catheter," filed April 4, 1997 and issued March 2, 1999, and EPO Publication No. EP 0 868 923 A2 entitled "Steerable Catheter with Tip Alignment and Surface Contact Detector," published March 31, 1999, are incorporated herein by reference in their entirety. These applications teach steerable catheters and methods of use, particularly adapted for PMR use. The distal portion of the catheters are deflectable. Rotation of the catheters, therefore, such as within the left ventricle during a PMR procedure, will allow treatment of essentially any surface area within the ventricle. The catheters have a relative movement compensation mechanism for maintaining positioning between the distal portion of the catheter and a functional device disposed therein. The deflectable portion of the catheter is non-deformable.

Another approach to catheter construction for PMR is described in International Publication WO 96/35469, entitled "System for Treating or Diagnosing Heart Tissue," International Application No. PCT/US96/06700, filed May 9, 1996 by Kesten et al., and WO 98/39045, entitled "Catheter with Three Sections of Different Flexibilities." International Application No. PCT/US98/04484, filed Mar. 6, 1998 by Javier et al., are also hereby incorporated by reference in their entirety. In these systems, an aligning catheter, shaped to extend along the long axis of the left ventricle, guides a laser catheter to various and predetermined individual points within the left ventricle. The intraluminal catheter has an elongated tubular shaft with proximal, intermediate, and distal shaft sections for positioning a therapeutic or diagnostic device within a patient's body region, such as a heart chamber. The intermediate shaft section has greater flexibility than the proximal or distal shaft sections, and is preferably of sufficient flexibility to easily assume the curvature of the patient's aortic arch, and reduce the force of contact between the distal end of the catheter and tissue defining the patient's body region to thereby reduce restriction on the rotation of the catheter. The flexible intermediate shaft section is preferably of a length to occupy a significant portion of the arctic arch, and the catheter overall length is preferably sufficient to have a catheter proximal extremity extending out of the patient and a distal extremity extending at least into an aortic passageway adjacent the patient's left ventricle. In certain embodiments, the distal section of a guiding or first delivery catheter, is provided with a double bend, or other predetermined geometry and dimension, to facilitate a perpendicular approach by a laser or other energy delivery device to the endocardial surface of the left ventricle.

Fluoroscopy is used during a PMR procedure to locate the distal tip of an energy delivery device inside the heart to ensure proper channel formation and prevent excessive penetration of the myocardial tissue of the heart. Typically, dye is injected into the chambers of the heart to provide contrast and enhance the fluoroscope image. There are problems associated with the use of fluoroscopy. The dye usually dissipates before all the revascularization channels and/or pathways of a PMR procedure can be created. Moreover, since most current fluoroscopy imaging systems are two-dimensional imaging systems, during a PMR procedure the cardiologist must continuously monitor two perpendicular planar images of a heart to more accurately determine the distal tip position of an energy delivery device in a three-dimensional perspective.

Additionally, tracking channels previously created during the procedure is another problem since the screen may not readily show locations of these previously created channels. Utilizing additional or excessive radiation to help track the distal tip of the energy delivery device or track previously created channels while using a fluoroscopic system poses radiation hazards to patient and operating room personnel.

U.S. patent 5,369,678 to Chiu, entitled "Method for tracking a catheter probe during a fluoroscopic procedure," teaches a fluoroscopy system for monitoring the location of a catheter inside a body during balloon angioplasty or laser ablation for percutaneous interventional procedures. Chiu teaches a method for determining catheter tip location from fluoroscopic images using digital imaging processing techniques that confine full X-ray dosage to a central area, compensating for the reduced X-ray dosage in the peripheral areas by computer imaging enhancement but does not solve the problems currently associated with the use of fluoroscopy for guidance and visual marking in PMR and other intracardiac catheter-based procedures.

It is desirable to have apparatus and methods that use integrated hardware of a processing system, video capture, image display and manipulation and angular feedback from the fluoroscopy device to assist the cardiologist during the PMR procedure. There is a further need for apparatus and method that solves the problems associated with the use of fluoroscopy for guidance and visual marking in PMR and other intracardiac catheter-based procedures. Furthermore, there is a need to reduce the complexity of positioning such catheters and estimating the precise position inside the left ventricle to form channels during myocardial revascularization.

To carry out a surgical procedure, with the use of a surgical device entering and moving within a human body, it is often necessary to ascertain the position and orientation of the distal portion of the surgical device to ensure proper placement of the distal portion during application of a desired therapy.

The present invention involves the tracking of an surgical device within a human body. Various input instruments provide position and orientation information with respect to the surgical device and pass this information along to a processing unit which then interprets the information through the use of certain algorithms. The information is then merged with additional data acquired and used as a basis for generating at least one output descriptive of the position and orientation of the surgical device.

Therefore, a first object of the present invention is to replace the current manual channel mapping technique with a computerized, video-based mapping technique having improved accuracy.

A further object of the present invention is to decrease the degree to which the fluoroscopy related PMR procedure is dependent upon additional visualization capability or means, thus allowing the user to remain predominantly in a single plane projection throughout most of the procedure.

Another object of the present invention is to provide for a more accurate depiction of a surgical device within the left ventricle of a heart by gathering data from different sources and then correlating the gathered data with a common point of reference, generating various outputs from the correlated data

Still another object of the present invention is to provide a system for alleviating a surgeon from performing complex interpretations of data perceived during a surgical procedure.

Yet another object of the present invention is to provide a system for allowing a surgeon to create or dictate a depiction of a surgical procedure, assisting the surgeon during a specific task as part of the surgical procedure.

Another object of the present invention is to assist a surgeon in the placement of a surgical device at a target tissue area during a surgical procedure, the surgeon being able to view a depiction of the surgical device in a live fluoro image.

Still another object of the present invention is to assist a surgeon in the placement of a surgical device at a target tissue area during a surgical procedure, the assistance being provided in a final indication of a target tissue site in a generated depiction based on an initial input from the surgeon descriptive of the target tissue site location in another depiction.

Numerous other advantages and features of the present invention will become readily apparent from the following detailed description of the invention and the embodiments thereof, from the claims and from the accompanying drawings.

In the drawings:

Fig. 1 is a block diagram of an enhanced tracking system in accordance with the present invention.

Fig. 2A is a perspective view of a geometric representation of the left ventricle portion of a heart.

Fig. 2B is a right anterior oblique projection view of an energy delivery device within the left ventricle of a heart in accordance with the present invention.

Fig. 2C is a left anterior oblique projection view of an energy delivery device within the left ventricle of a heart in accordance with the present invention.

Fig. 3A is a right anterior oblique project view of a heart.

Fig. 3B is a left anterior oblique projection view of a heart.

Fig. 4A is an elevation view of a first energy delivery device used in accordance with the present invention.

Fig. 4B are representative top, orthogonal and elevation views of an exemplary radio opaque marker band for use on the distal tip of an energy delivery device in accordance with the present invention.

Fig. 4C is a representative RAO-30 projection view of an intraventricular energy delivery device in the left ventricle in accordance with the present invention.

Fig. 4D is another representative RAO-30 projection view of an intraventricular energy delivery device in the left ventricle in accordance with the present invention.

Fig. 4E is a representative elevation view of the distal end of a second energy delivery device in accordance with the present invention.

Fig. 4F is a representative elevation view of the partially deflected, apically oriented distal end of a second energy delivery device in accordance with the present invention.

Fig. 4G is a representative elevation view of the partially deflected, laterally oriented distal end of a second energy delivery device in accordance with the present invention.

Fig. 4H is a representative elevation view of a partially deflected third energy delivery device in accordance with the present invention.

Fig. 4I is a RAO and LAO projection view of the distal end of another energy delivery device within the left ventricle of a heart.

Fig. 4J is another representative RAO and LAO projection view of the distal end of another energy delivery device within the left ventricle of a heart. Fig. 5A is a representative RAO-30 projection view of another energy delivery device within the left ventricle of a heart.

Fig. 5B is a representative LAO-60 projection view of another energy delivery device within the left ventricle of a heart.

Fig. 6 is a representative RAO-30 projection view of the distal end tip of an energy delivery device directed perpendicularly towards internal surface areas of the left ventricle.

Fig. 7 is a series of representative RAO-30 projection views of a series of channels formed by the distal end tip of an energy delivery device directed perpendicularly towards internal surface areas of the left ventricle.

Fig. 8 is a series of representative LAO-60 projection views of a series of channels formed by the distal end tip of an energy delivery device directed perpendicularly towards internal surface areas of the left ventricle.

Fig. 9 is a set of flowcharts defining a preferred method performed in accordance with the present invention.

Fig. 10 is an exemplary view of an output generated in accordance with the present invention.

It will be understood while numerous preferred embodiments of the present invention are presented herein, numerous of the individual elements and functional aspects of the embodiments are similar. Therefore, it will be understood that structural elements of the numerous apparatus disclosed herein having similar or identical function may have like reference numerals associated therewith.

Additionally, it should be generally understood that the following discussion, while directed to a PMR procedure specifically, would apply to any surgical procedure involving the tracking of surgical devices, including position and orientation, within a body, or near or within a target tissue site.

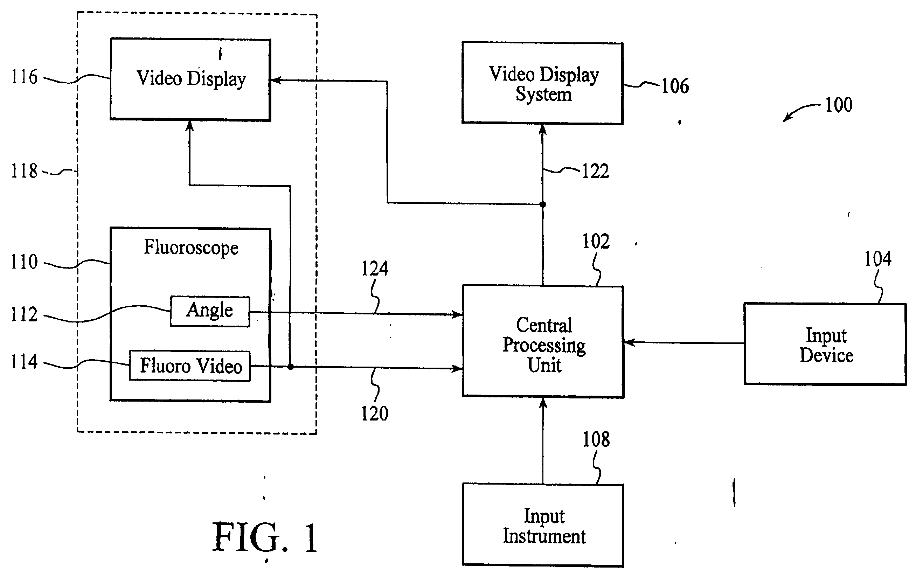

Now referring to Figs. 1, 2A, 2B, 3A, 4A, 6, 7, and 8, a PMR procedure performed in accordance with the present invention can be more readily understood. Revascularization channels are formed using energy delivery systems which apply energy to specific target tissue sites within the left ventricle of a heart, ablating or otherwise causing an injury at the site. The energy is transmitted from a point external to a patient's body, to the target tissue site, by an energy delivery device. Such energy delivery systems include, but are not limited to, the single use, or use in combination, of one or more of the following energy sources: laser, radio frequency (RF), ultrasound, fluid, thermal, mechanical systems including piercing, coring, and other systems requiring translational or rotational movement, or any other system which incorporates an energy source and transmits energy through an energy delivery device to a target tissue. The specific configuration of the energy delivery device is dependent on the energy being transmitted.

It is also contemplated that the present invention can be used in conjunction with energy delivery devices involved with other medical therapies or additional tools including one or more functional devices. For example, an energy delivery device may comprise a drug delivery device where the operational aspects of the drug delivery, including the timing and volume of a delivered dosage with respect to energy delivery, are controlled by a processing system, in accordance with the invention described herein. Drug delivery devices, by way of example only, include those disclosed in International Publication No. WO 99/49773, entitled "DELIVERY CATHETER SYSTEM FOR HEART CHAMBER," published October 7, 1999, U. S. Patent 5,999,678, entitled "LASER ASSISTED DRUG DELIVERY," filed December 17, 1996 and issued December 7, 1999, and U.S. Patent 5,925,012, entitled "LASER ASSISTED DRUG DELIVERY," filed December 27, 1996 and issued July 20, 1999, all three incorporated herein by reference.

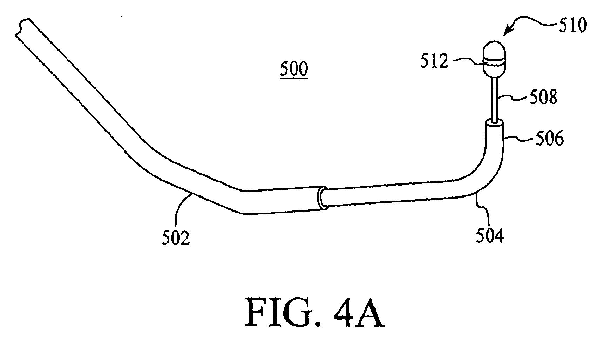

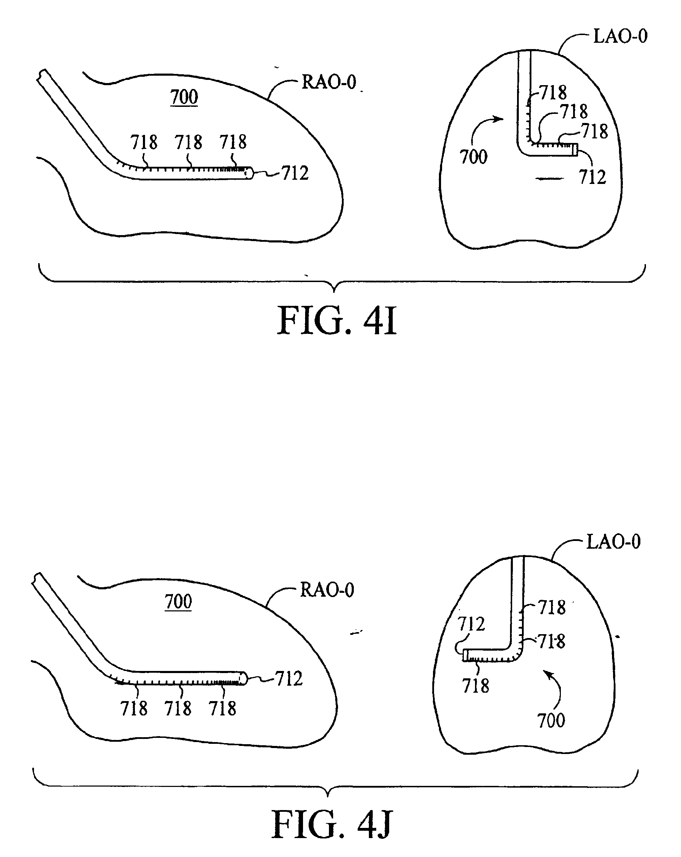

With specific reference to Fig. 4A, one type of energy delivery device which may be used in accordance with the present invention is shown in the form of a laser catheter 500. Catheter 500 is a coaxial catheter system as taught by Kesten et al. (International Application No. PCT/US96/06700) and includes an aligning catheter 502 and a laser catheter 504 having a preformed distal tip 506. Laser catheter 504 is rotatably and slidably disposed within the aligning catheter 502. At least one optical fiber 508 exits the catheter 504 at the distal tip 506 opening and is rotatably and slidably disposed within the laser catheter 504. A lens portion 510 is operably attached to the distal tip of the optical fiber or fiber bundle 508 focusing laser energy towards a target tissue site. Attached to the lens portion 510 is a cylindrical radio opaque marker 512. As is discussed in more detail below, additional markers attached to catheter 500 may be used to help better determine the position and orientation of catheter 500 within a chamber of the heart.

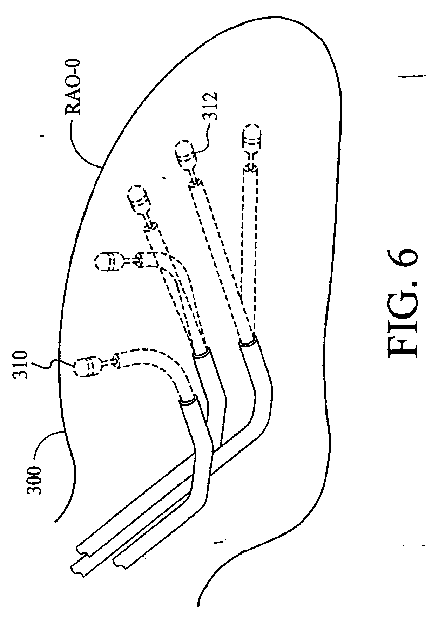

During a PMR procedure, catheter 500 is inserted into, for example, the femoral artery at the groin and advanced through the vasculature toward the heart, the distal portion of the catheter 500 passing through the aortic arch and into the left ventricle of the heart. Once in the ventricle, the catheter 500 is guided to the endocardium where the distal tip 510 of the catheter 500 transmits energy from a source to the target tissue, the transmitted energy creating pathways through the endocardium and a portion of the myocardium. Fig. 6 depicts catheter 500 in use during a PMR procedure showing a small representation of angular combinations achievable providing a generally perpendicular approach to a specific target tissue site.

As the catheter 500 is advanced toward and then within the left ventricle of the heart and directed toward a target tissue site, the radio opaque marker is viewable on the video display 116 of the fluoroscopy system 118. The live fluoro image on video display 116 assists the surgeon in advancing or navigating the catheter 500 toward the target tissue site, as well as positioning the distal tip 510 at the site.

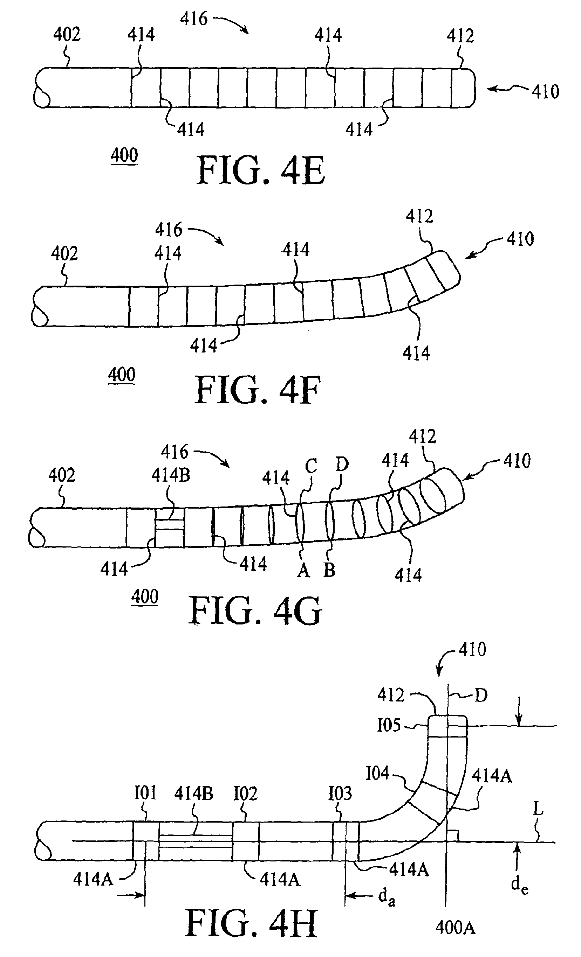

With reference to Fig. 4E another energy delivery device, catheter 400, is shown. Catheter 400 has a main shaft portion 402, and a distal end 410 comprising a distal tip radio opaque marker 412 similar to the marker 512 of catheter 500. Catheter 400 also comprises a plurality of thin circular markers 414 spaced a predetermined distance from each other along a distal portion 416 of catheter 400 defined by the distal end of the main shaft 402 and the marker 412. The distal portion 416 of catheter 400 also comprises a deflection mechanism which deflects the distal portion, the central axis of an opening at the distal end 410 and a central axis of the main shaft 402, where the shaft 402 interfaces with the most proximal marker 414, forming an angle therebetween. Alternatively, as shown in Fig. 4G, the markers 414A can be constructed to have similar dimensions as marker 412, each marker 414A being a predetermined distance from one another.

For purposes of discussion, energy delivery device 300 can be any energy delivery device having the common attributes of a distal tip 310 comprising at least one radio opaque marker and at least one lumen. Thus, energy delivery device 300 can be catheter 400, catheter 500, or any other device similar in shape used as or in combination with surgical tools during a surgical procedure, and having a structure similar to device 300 as described immediately above.

Now with specific reference to Fig. 1, the guiding of an energy delivery device from the femoral artery to the left ventricle can be better understood. A fluoroscopy system 118, shown in dashed, includes a fluoroscope 110 and at least one video display 116. Fluoroscope 110 is a device well known in the art, which includes an angle sensor 112 and a fluoro video source 114.

As in other systems, fluoroscope 110 further includes an X-ray source and an opposing image detector (not shown). Typically, during use, a subject is placed between the X-ray source and the image detector. After the X-ray source is activated, X-rays detected by the image detector define an image which is then encoded into a video signal. The encoded video signal is then provided by the fluoroscope 110 as an output, as part of fluoro video source 114. During a PMR procedure, the X-ray source and opposing image detector are moved to provide views of the subject from different perspectives, each perspective being defined by a plane having a specific orientation with respect to the subject. The Fluoroscope 110 can be a single plane or a bi-plane unit, the single plane unit being able to provide a single image with respect to a single orientation plane while the bi-plane unit is able to simultaneously provide two separate images, each image corresponding to a separate orientation plane. A video signal from the fluoro video source 114 to be displayed on video display 116 is typically referred to as live fluoro since the video signal represents real-time views of the subject.

It should be apparent, given a bi-plane fluoroscope 110, that the fluoroscopy system 118 may include a second video display 116a (not shown), where a first image corresponding to a first orientation plane of the subject is displayed on the first video display and a second image corresponding to a second orientation plane of the subject is displayed on the second video display. Alternatively, where the video display 116 is of sufficient size, both images may be displayed on the single video display 116.

U.S. Patent Application 09/107,843, entitled "Intracorporeal Device with Radiopaque Marker", filed by Rosenthal et al. on June 30, 1998, is incorporated herein by reference in its entirety. This application teaches an energy delivery device compatible with the present invention in the form of a catheter with an elongated shaft having at least one asymmetric radio opaque marker disposed upon or within the distal end thereof. The radio opaque marker enables the user to distinguish orientation of the distal end of the device using a fluoroscopy system.

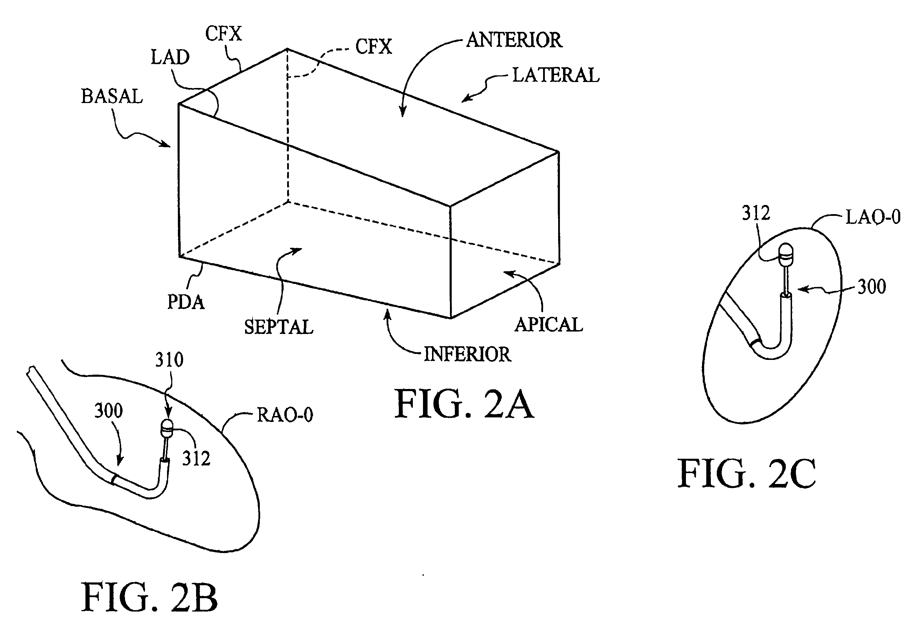

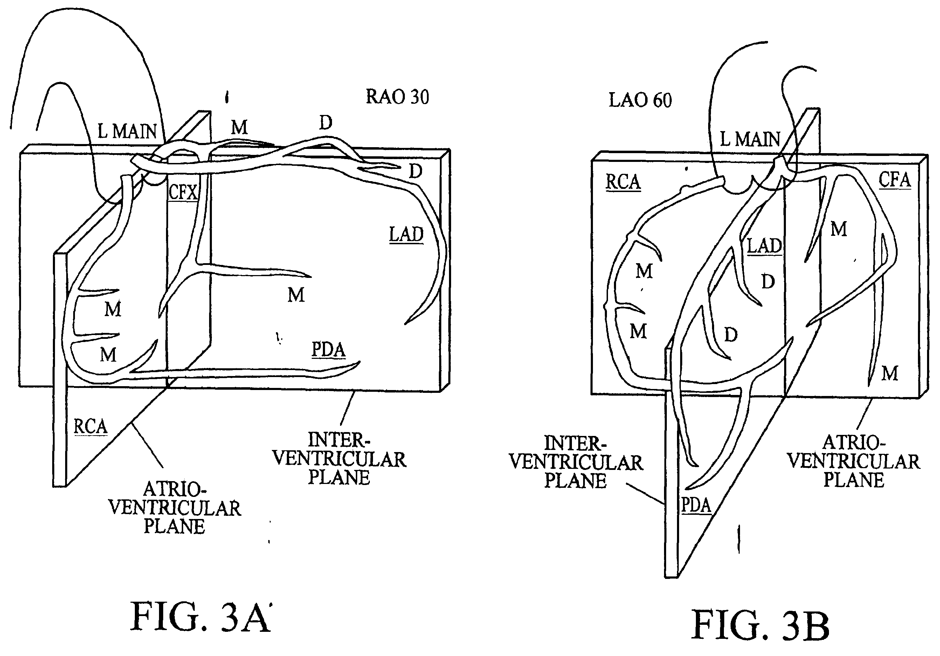

Now referring to Figs. 2A and 3, a geometrically depiction of the general structure of the left ventricle of a heart, generally indicating the left ventricular planar surfaces and the associated coronary vasculature is shown. For example, the anterior planar surface is generally defined by the left anterior descending (LAD) coronary artery. As is shown in Fig. 3A, the LAD coronary artery generally defines the anterior surface and, at its most distal end, the apical surface of the left ventricle while the PDA of the right coronary artery (RCA) defines the inferior surface of the left ventricle. As is shown in Fig. 3B, the right coronary artery (RCA) and the circumflex (CFX) of the left coronary artery define the septal and lateral surfaces of the heart, respectively.

Also shown is the posterior descending artery (PDA) of the right coronary artery (RCA). Figs. 2B and 2C are representative right anterior oblique (RAO) and left anterior oblique (LAO) views of an energy delivery device within the left ventricle of the heart of Fig. 2A, respectively.

While the views of Figs. 2B and 2C are referred to as RAO and LAO views, respectively, more specifically, the view of Fig. 2B is referred to as RAO-30 since the view is 30° from vertical, and the view of 2C is referred to as LAO-60 since the view is 60° from vertical. For simplicity, hereinafter RAO refers to RAO-30 and LAO refers to LAO-60, unless stated otherwise.

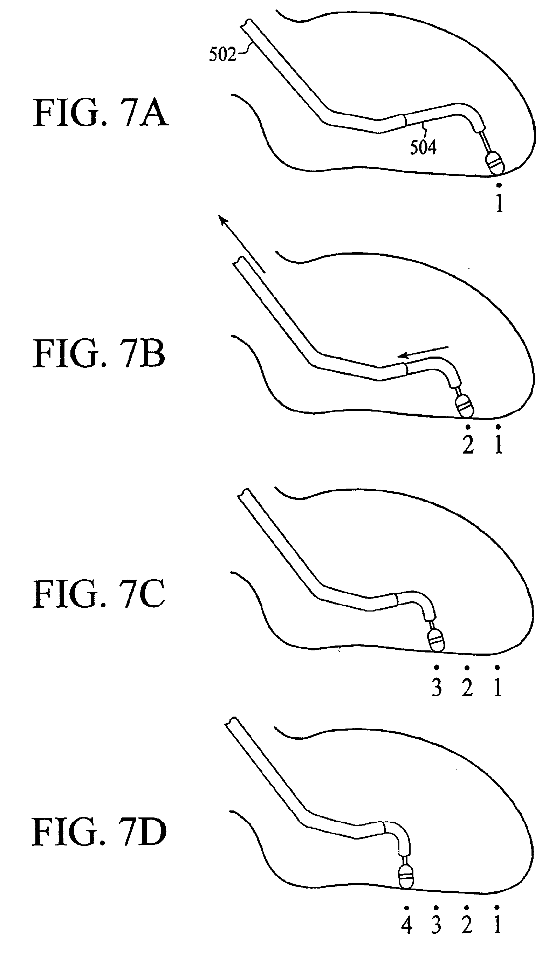

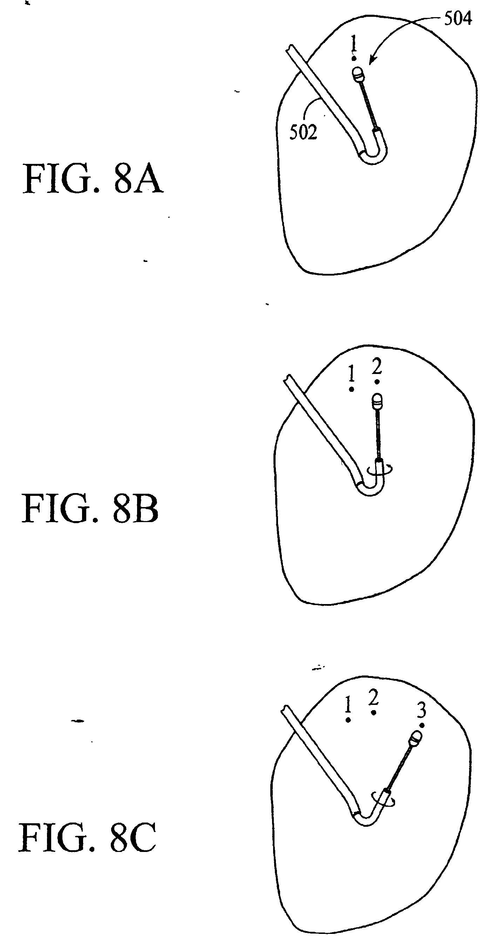

As is shown in Figs. 2A and 2B, the RAO view is generally parallel to the longitudinal axis of the left ventricle, generally referred to as the interventricular plane. As shown in Figs. 2A and 2C, the LAO view is generally parallel to the minor axis of the left ventricle, generally referred to as the atrioventricular plane. With reference also to Figs. 3A and 3B, the relationship between the interventricular and atrioventricular planes and the cardiac vasculature of the left ventricle can be better understood. Fig. 3A is a representative RAO view generally parallel to the interventricular plane and, thus, perpendicular to the atrioventricular plane. Now with reference to Figs. 7 and 8, two illustrative revascularization channel creation techniques are shown. Fig. 7 generally depicts an RAO view of the heart with channels being formed with the use of an energy delivery device similar to catheter 500. Starting from a point more near the apex of the heart a first channel 1 is created as shown in Fig. 7A. The laser catheter 504 is then retracted and a second channel 2 is formed as shown in Fig. 7B. This process is repeated to create channels 3 and 4 in Figs. 7C and 7D, respectively.

Fig. 8 depicts a second procedure utilizing the features of catheter 500. After a first channel 1 is created as shown in Fig. 8A, the laser catheter 504 is rotated within aligning catheter 502 to reposition the distal tip 510 to a new target tissue site 2, as shown in Fig. 8B. After channel 2 is created the laser catheter is once again rotated to reposition distal tip 510 and a third channel 3 is created as shown in Fig. 8C.

Generally, the present invention involves the use of a processing system to track and provide a representative output of the relative position and orientation of a surgical device, such as an energy delivery device, with respect to its surrounding environment and other data acquired during a surgical procedure, such as a PMR procedure. Tracking of the surgical device is important since it allows a surgeon to associate events occurring during the surgical procedure with respect to the position and orientation of the surgical device. For example, during a PMR procedure, tracking of the distal tip of an energy delivery device allows for the mapping of created revascularization channels, ensuring that such channels are accurately created at specific target tissue sites and are separated a predetermined distance. Additionally, tracking, in accordance with the present invention, allows a surgeon to initially select a target tissue site from a display, at least partially created by algorithms executed by a processing system, and then navigate to and align the distal tip of the energy delivery device with the selected target tissue site.

An energy delivery device used in accordance with the present invention is adapted to accept one or more sensors specifically designed and placed on the energy delivery device in a known arrangement. The term sensor as used herein is meant to mean any device that receives and responds to a signal or stimulus, either actively or passively. A radio opaque marker, when used with a fluoroscopy system, is deemed to be within the definition of a sensor as used herein. Additionally, passive devices such as magnets, and transducers such as accelerometers, pressure sensing devices, and ultrasound devices, are also deemed to fall within the scope of the term sensor as used herein.

As stated just above, these sensors can be active or passive devices. For example, as mentioned above, one such sensor may be a specifically designed radio opaque marker which, when subjected to X-rays from a fluoroscopy system, absorb such X-rays providing contrast between the marker and its surrounding environment, allowing the marker structure to become visible when displayed on an associated fluoro display. These markers can then be analyzed to determine the position and orientation of the energy delivery device, to which the marker is attached.

While a radio opaque marker is preferably used at the catheter tip to allow for aspect perspective, no specific design of radio opaque marker is preferred as long as the specific dimensions defining the marker are provided to the processing system. The particular design aspects of the markers, on the other hand, are directed to gaining from a single view some or all of the information that might have been obtained from a pair of orthogonal views. This is accomplished first by using radio opaque elements designed to present additional information regarding the position and orientation of the distal tip of an energy delivery device when viewed from a single fluoroscopic projection, and then combining such radio opaque elements with additional information acquired or generated by the processing system, as is discussed in more detail below.

A magnet is another example of a passive sensor which may be used in accordance with the present invention. In contrast, a sensor can be an active device which transmits a signal in response to receiving or otherwise directing a signal in the direction of the element.

Various input instruments are used to track the elements, providing the processing system with position and orientation data in one of several different formats. The data is processed by the processing system and provided in a more usable form to the surgeon performing the surgical procedure. Output formats include two and three-dimensional output depictions of channel formation and computer generated graphical representations of different views of the ventricle showing the energy delivery device therein, both being able to be incorporated with the live fluoroscope images as seen by the surgeon performing the procedure, as a video overlay which is combined with the live fluoroscope images or as a separate image in close proximity to the fluoroscope images allowing the surgeon to easily view both in his field of view.

Referring now to Fig. 1, a general embodiment of an enhanced tracking system in accordance with the present invention can be more readily understood. An enhanced tracking system 100 includes an input instrument 108, a CPU 102, and a video display system 106. To assist a surgeon during a PMR procedure the fluoroscopy system 118, as initially described above, is typically utilized.

The input instrument 108 provides the CPU 102 with raw data with respect to the position and orientation of an energy delivery device during a surgical procedure. The raw data, along with additional data acquired, provides the CPU 102 with information to generate at least one system output, as described in more detail above. The raw data can be in the form of any signal described herein with reference to the data acquisition system of CPU 102. It is important to note that the input instrument 108 can operate alone or in combination with other devices to provide CPU 102 with requisite information for generating system outputs.

Therefore, fluoroscopy system 118, one or more input devices 104, or a combination of the two, can be considered an input instrument 108 used in accordance with the present invention. By way of example only, input instrument 108 includes, but is not limited to, a live fluoro video signal provided by a fluoroscope depicting a surgical device having at least one sensor attached thereto, a live fluoro video signal as described immediately above in combination with one or more input devices 104, a system utilizing magnetic fields generated by one or more magnetic sensors located on a surgical device, any other system which can provide position and orientation information of a surgical device, or any combination of the examples above.

CPU 102 can be any programmable computing device having a microprocessor of sufficient efficiency such that the processes described herein are able to be performed within predetermined time limits, a memory module of sufficient size for the temporary storage of program and acquisition data, and storage devices for temporary and non-volatile storage of program and acquisition data (not shown). CPU 102 can be, for example, a personal computer system or computer workstation, able to provide functionality as described herein. A storage device for non-volatile storage may include one or more hard disk drives or a more portable storage media.

CPU 102 further includes a data acquisition system (not shown) capable of receiving various input signals and converting such signals into a form more compatible for analysis by CPU 102. Such received signals include analog signals, such as certain video signals or other varying voltage signals, digital signals such as switches handled as binary inputs, or any other signal capable of being described in terms of current or voltage. For example, while the source of a signal may be a transducer measuring a certain phenomena, the conditioned signal provided to the acquisition system may be a voltage which is proportional to the unit-phenomena measured. The waveform of an electrocardiogram (ECG) or an oscillating signal representative of a patient's respiratory cycle are two examples of such signals.

CPU 102 includes, as part of its data acquisition system, at least one, preferably two, video frame grabbers. The frame grabber continuously receives a video signal in a known format, such as a standard NTSC or PAL signal, and, upon command, digitizes the current video frame being received. Additionally, the video signal provided to the CPU 102 could be a digital signal provided by the fluoroscopy system 118.

It is important to note that the CPU 102 could have several frame grabbers, each frame grabber having a corresponding video signal as an input, thus enabling the CPU 102 to receive and digitize several video signals, simultaneously. The digitized video frame or frames can then be stored for analysis and/or immediately displayed. Therefore, since the frame grabber has the ability to freeze or otherwise grab and digitize a frame of video upon command, the digitized frame can be correlated with time, and each digitized frame from a plurality of frame grabbers will represent a specific view at one instant in time.

In a preferred embodiment and as discussed more fully below, the frame grabber includes additional inputs from which to correlate with the video data. Thus, for example, as a video frame is digitized, other known parameters regarding the patient such as current cardiac cycle data and current respiratory cycle data can be acquired and stored with the frame for later analysis.

CPU 102 also includes a video combiner and at least one video output port which interfaces to the video display system 106 such that video signals generated in response to data acquired during a surgical procedure can be combined with other video signals such as the live fluoro views. The video combiner can be performed by hardware or software if requisite processor bandwidth is available. Display system 106 includes at least one video display to display the output video signal from the video output port of CPU 102.

The data acquisition system of the CPU 102 also receives an input signal from at least one input device 104.

The input device 104, as is described in more detail below, provides CPU 102 with data and/or other event markers from which a correlation can be made with additional acquired data, such as video signals or discrete signals such as the activation of an energy source to be transmitted to a target tissue site by an energy delivery device. Therefore, input device 104 may be any suitable event marking device such as a switch, or may provide analog or digital data regarding the surgical procedure being performed such as signals received from a pointing device, or a combination of the two. Pointing devices contemplated herein include, but are not limited to, a computer mouse, trackball, light pen, keyboard, or any other device which can define a point in at least two-dimensional space, working alone or in combination with another structure such as a video display. Alternatively, input device 104 could be a switch whose signal is acquired by CPU 102 and defining an event which then provides a basis for further data analysis, as described in more detail below.

Based on input data provided by at least one input instrument 108 and at least one input device 104 during a surgical procedure, each of which may also define surgical procedural events, CPU 102 can process the data with reference to the events acquired and display desirable information in response to the events in real-time or near real-time during the procedure. The displayed information can be displayed on one or more video displays as part of video display system 106 and/or on one or more live fluoro video displays, as discussed below.

The surgeon performing the surgical procedure can then use this displayed information to track progress or more accurately position surgical instruments utilized during the surgical procedure. For example, during a PMR procedure, as is fully discussed below, revascularization channels created by a surgeon can be tracked and mapped in an efficient manner providing the surgeon with a level of certainty that the channels are being created at a desired location and a predetermined distance from each other within the left ventricle. Further, the displayed information can be used in real-time to assist a surgeon in placing the distal tip of an energy delivery device at the desired target tissue site.

Prior to performing a PMR procedure, the CPU 102 acquires two virtual two-dimensional depictions of the left ventricle, sometimes referred to as ventriculograms or V-grams. While the two-dimensional depictions or V-grams can be created from any two non-parallel planar perspectives, the two-dimensional depictions are preferably created from the RAO and LAO views. The angle sensor 112 of the fluoroscope 110 provides the CPU 102 with an angle relationship between the two planar perspectives. It should be clear, therefore, given the angle relationship, a point object in each perspective can be defined as the intersection point of two lines, a first line normal to the first planar perspective, and a second line normal to the second planar perspective.

A radio opaque dye is injected into the left ventricle. Since the radio opaque dye absorbs X-ray radiation, the dye will create a contrast between the surrounding tissue of the left ventricle and the left ventricle chamber itself. The resulting fluoroscope image is provided to CPU 102 for acquisition. In a preferred embodiment of the present invention, the major features of the acquired heart image, such as walls and coronary vasculature, are also outlined using input device 104, such as a computer mouse or light pen. The ventricle outline acquired, and coronary vasculature if desired, is then overlaid onto a corresponding live fluoro display providing the cardiologist information regarding the ventricle structure after the dye has dissipated.

More specifically, with an RAO view being displayed and using the input device 104, the cardiologist outlines the left ventricle wall structure as defined by the contrast developed by the dye, defining the RAO view of the left ventricle chamber. The cardiologist outlines the left ventricle wall by identifying several points along the dye-defined chamber wall. The points are identified by manipulating the input device 104 to the edge defined by the dye and heart tissue and sending a command to CPU 102 to acquire and define that point. Once an adequate number of points have been defined, typically 16-20, the CPU 102 is commanded to connect the individual points with a smooth line, using interpolation techniques, resulting in a well defined left ventricle chamber with respect to the RAO view. In a similar fashion, with an LAO view being displayed, the above procedures are performed again to define the left ventricle chamber with respect to the LAO view.

Outline views RAO-O and LAO-O as shown in Figs 2B and 2C, respectively, are exemplary resultant views as processed by CPU 102 based on inputs provided by the cardiologist. Once acquired, the outline views are then stored in CPU 102 for later use during the surgical procedure. As is discussed in more detail below, the outlines obtained of the left ventricle above can be provided as an overlay to a live fluoro video signal by the combiner of CPU 102. The combined image is then provided to video display 116 such that, after the radio opaque dye dissipates, the overlay provides structural definition which would otherwise not be visible. If a second video display 116a is utilized, a combined image corresponding to the RAO view could be displayed on video display 116 while a combined image corresponding to the LAO view could be displayed on video display 116a.

Alternatively, with a video display 116 of sufficient size, both, RAO and LAO, combined images could be displayed on a single video display 116. Furthermore, if it is desirable to display live fluoro separate from combined images then live fluoro could be displayed on video display 116, 116a and the combined images could be displayed on one or more video displays as part of video display system 106. Each monitor 116, 116a and video monitors of the video display system 106 have a "last image hold" function, if applicable, to allow the user to simultaneously compare one or more video images. The last image hold function can also be performed by the CPU 102. In this latter arrangement, the CPU 102 can hold or freeze one image with respect to another image when both images are part of a single video signal provided to a video monitor for viewing.

In addition to the V-gram, an angiogram can be performed by injecting radio opaque dye into the heart vasculature. Using outline imaging techniques described above, the angiogram could be used to estimate the outer surface of the ventricle while the V-gram acquired above provides an estimation of the inner surface and, therefore, an estimation of the heart wall thickness can be made. If only single plane data is collected, then the myocardial thickness in that plane can be estimated. Alternatively, if data is collected from multiple views (using either a bi-plane or a single plane fluoroscopy system), then the myocardial thickness with respect to the two views, such as RAO and LAO views, could be estimated.

As is more fully discussed below, in a more preferred embodiment, the V-gram and angiogram outlined images are obtained through the use of video signal digitization and acquisition of the digitized signal by the CPU 102. CPU 102 acquires the outline image definitions using edge-detection algorithms such that when the radio opaque dye is injected into the left ventricle the contrast between the dye within the ventricle and the ventricle wall is perceived. As discussed above relative to the manually obtained outline images, the outline images acquired by the fluoro video is stored for later use during the surgical procedure.

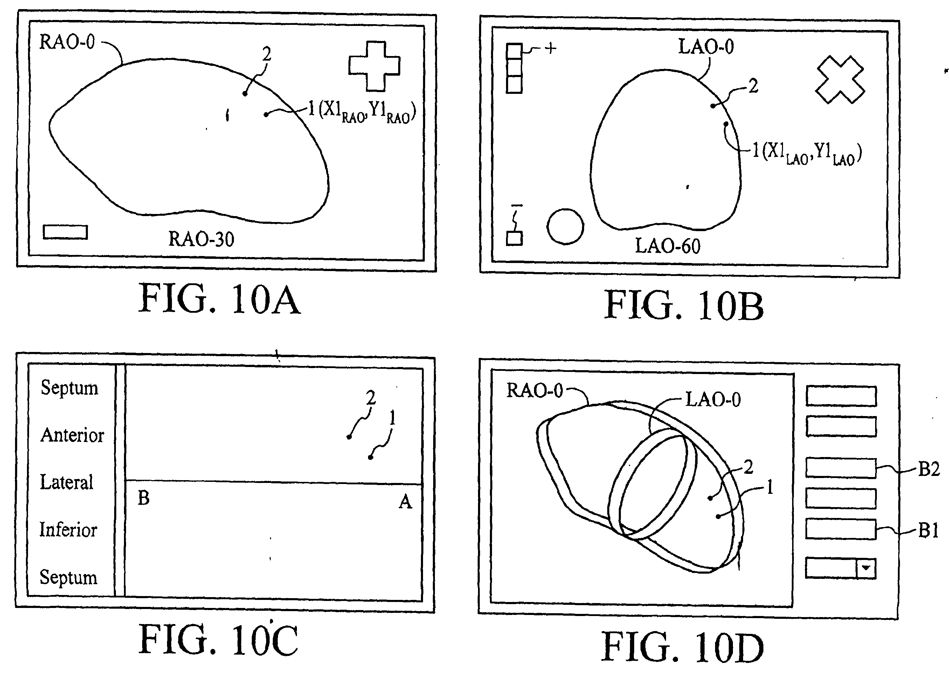

With reference now to Fig. 10, output depictions generated by the present invention can be more readily understood. Fig. 10 is a representative view of several outputs generated by the tracking system as displayed on a single monitor. As stated above, while the output depiction is shown on one display, several displays 116a could be used to display the output information. With specific reference to Figs. 10A and 10B, RAO and LAO combined images are shown. During a surgical procedure, as discussed above, the CPU 102 digitizes live fluoro images resulting in working images. A working image is defined or created for each video input corresponding to a particular view, an RAO or LAO view for example. During methods in accordance with the present invention certain algorithms are executed by CPU 102 resulting in the depiction of position and orientation information of a portion of the energy delivery device (not shown) being merged with other information, such as the stored outline images RAO-O and LAO-O and target tissue sites, shown as 1 and 2 in Fig. 10, defining the location of an application of a desired therapy, into the corresponding working image. This is generally referred to as therapy mapping and , more specifically, channel mapping. The working image is then overlaid onto the live fluoro video of the corresponding view by the combiner and displayed. The outline views, RAO-O and LAO-O, are representative RAO-30 and LAO-60 views obtained as described above.

The symbols "+", "-", "X", and "O" are used by CPU 102 to align the current working image with the live fluoro video, as is described in more detail below. This helps to compensate for any rotational movement of the patient with respect to the fluoroscopy system 118.

In a more preferred embodiment, the LAO view is generated in its entirety by CPU 102 allowing for a more expeditious procedure when only a single-plane fluoroscope 110 is available.

Now with reference to Fig. 10C and Fig. 2A, another output generated by the present invention is shown. Fig. 10C depicts an unfolded two-dimensional view of the left ventricle of the heart having the centerline of the lateral surface along the longitudinal axis of the left ventricle of the heart defining the center line or central axis of the view, starting from the basal surface, labeled B, on the left and leading to the apical surface, labeled A, on the right. This particular output provides a more simplistic view of the heart reducing the time which a surgeon uses in interpreting live fluoro or other more complex images of the target tissue site.

In comparison with the depiction of target tissue sites 1 and 2 of Figs. 10A and 10B, Fig. 10C shows site 1 more apical and more inferior than site 2. Under certain circumstances, target tissue sites may seemingly overlap each other in a specific view. The surgeon must then continuously scan the views, in this case the RAO and LAO views, to determine the spacing between target tissue sites. In contrast, only the view of fig. 10C is needed to ensure proper location of the therapy sites.

Also, as is described in more detail below, with an input device 104, the surgeon can define a site within the two-dimensional view of Fig. 10C and the CPU 102 will provide approximations of the target tissue site as part of working images to be combined with live fluoro. The surgeon can then guide the distal tip of the energy delivery device to the desired site and apply the desired therapy, ensuring that the site is at a predetermined location with respect to other established sites.

Another generated output in accordance with the present invention is shown in Fig. 10D. Fig. 10D provides a wire frame depiction of the left ventricle created by the CPU 102 from the outline images RAO-O and LAO-O and defined target tissue sites. The outline views are referenced to the interventricular and atrio-ventricular planes as shown in Figs. 3A and 3B and discussed above.

The three-dimensional depiction of Fig. 10D can be manipulated by the surgeon or an assistant during a surgical procedure by providing commands to CPU 102 via the input device 104, such as a keyboard or computer mouse. Thus, the surgeon can rotate the depiction about any axis and view the orientation of the target tissue sites. In a more preferred embodiment, the depiction would include an image representative of a distal portion of an energy delivery device within the left ventricle (not shown for clarity) allowing the surgeon to more accurately advance towards and place the distal tip of the energy delivery device at a desired target tissue site.

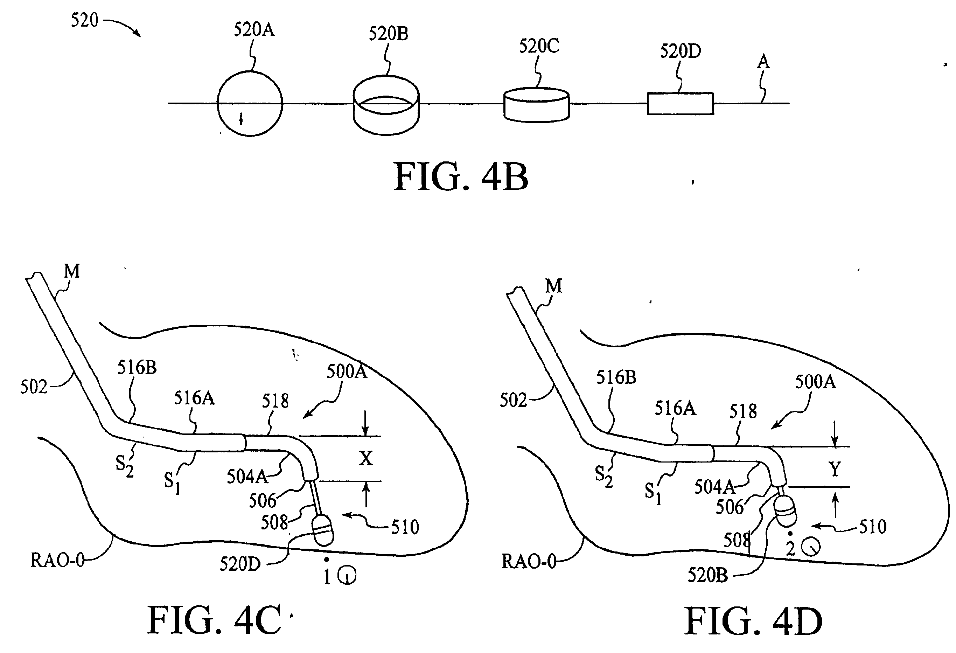

With reference to Figs. 4C and 4D another generated output in accordance with the present invention is shown. As is discussed in more detail below, one embodiment of the present invention utilizes a single view, an RAO view for example, and derives either a generated LAO view or a clock face image representative of the orientation of the distal tip of a specific energy delivery device. Using the clock face analogy, the LAO depicted view would show the anterior region of the heart from about 11:00 to 2:00, the lateral region from 2:00 to 5:00, the inferior region from 5:00 to 8:00, and the septal region from 8:00 to 11:00. As is shown in Fig. 4C, the energy delivery device is the catheter 500 oriented such that the aligning catheter 502, the laser catheter 504, the fiber optic or fiber bundle 508 are all parallel to the RAO plane of view. Therefore, in the LAO view, the depiction of the catheter 500 could be made with a single vertical line.

The clock face 1 depicts the orientation of the distal portion of the energy delivery device, and in this case, the laser catheter 504, the fiber optic or fiber bundle 508, and the distal tip 510, where the center of the clock face depicts the main axis of the laser catheter 504. Therefore, as shown, clock face 1 comprises a circle having a vertical arrow extending from the center of the circle to the outer surface, the vertical arrow depicting the orientation of the catheter 500 in the LAO view being parallel to the RAO plane of view.

In contrast, with reference to Fig. 4D showing the catheter 500 distal tip rotated towards the lateral surface of the left ventricle, the clock face 2 depicts the LAO view of the distal tip of catheter 500 pointing into the RAO plane view. Thus, a surgeon or other person present during the surgical procedure has a depiction of the distal tip orientation while looking at the single RAO view.

Additionally, a clock face depiction can be provided for each treatment location using methods described in detail below. Each clock face depiction representative of a treatment location can be viewed continuously or at the command of a user, such as the surgeon, during a procedure, by selecting the channel site or location with an input device for example.

Also, in accordance with the present invention, CPU 102 can provide a plurality of system signals related to the surgical procedure and displayed via indicators such as light emitting diodes devices on a display as part of video display system 106 or video display 116 or as graphical images merged into the working image and combined with a video signal and displayed on video displays, as described herein.

These system signals may be discrete or binary signals representative of patient or surgical procedure status, such as alarms preventing the application of a desire therapy more than once at a given target tissue site. Additionally, these system signals may also be related to the tracking system itself providing an indication that the system is not ready to acquire data, for example, or displaying system errors. Also, these system signals may involve the interaction between the tracking system and the surgical procedure. For example, if the energy delivery device is a laser fiber used to transmit laser energy to a target tissue site, it may be desirable to have the tracking system control the energy source such that the energy source is activated and energy is transmitted through the laser fiber only under certain conditions relative to cardiac or respiratory system cycles. The tracking system could provide an indication that the energy source is under its control.

It should be clear, while outputs are described separately above, the actual output from CPU 102 can comprise one or more of the outputs described above, in combination. As also stated above, it is important to note while these examples pertain to a PMR procedure specifically, it is apparent that such a system can be used in the placement of other surgical instruments and/or the application of materials such as various drugs or therapeutic agents, saline or other fluids, or other substances as described in U.S. Patent No. 5,840,059, entitled "Therapeutic and Diagnostic Agent Delivery", incorporated by reference in its entirety.

With reference to Fig. 1, a first embodiment of an enhanced tracking system in accordance with the present invention, incorporating features described above pertaining to the general embodiment, can be more readily understood. As stated above, the tracking system 100 includes the input instrument 108, the CPU 102, and the video display system 106. To assist a surgeon during a PMR procedure the fluoroscopy system 188, incorporating a fluoroscope 110 and one or more video displays 116, is typically utilized. Also, fluoroscopy system 118 provides guidance in placing the distal tip of an energy delivery device, which incorporates at least one sensor, at a target tissue site. In this first embodiment, fluoroscopy system 118 also acts as part of input instrument 108, providing, in part, position and orientation information regarding the energy delivery device.

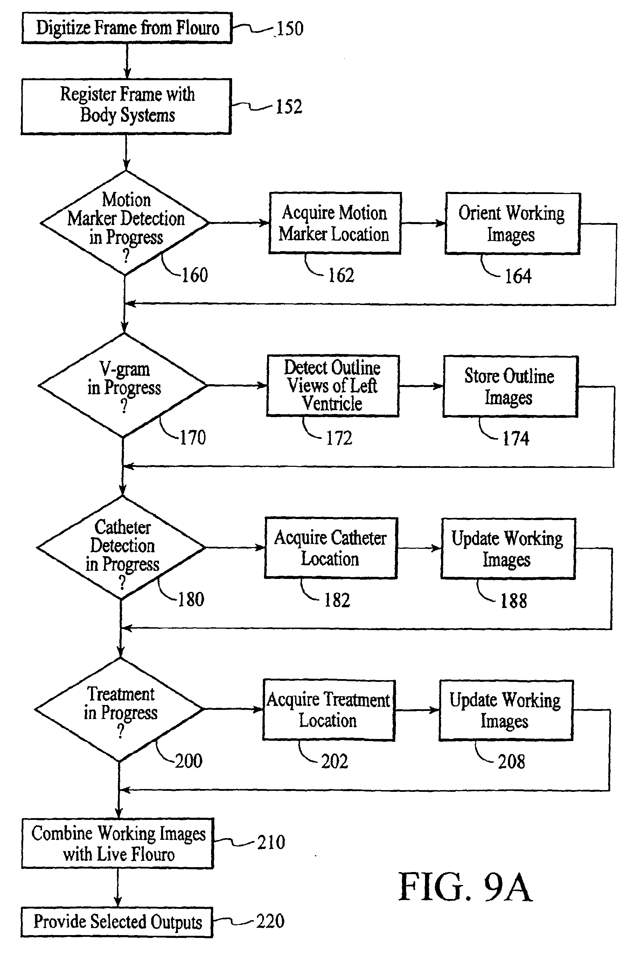

With reference also to Fig. 9A, a PMR procedure in accordance with tracking system 100 will be discussed. Fig. 9A is a representative flowchart of a preferred method performed in accordance with the first embodiment of the present invention. During the performance of the preferred method one or more fluoro images are digitized into working images. Other data are then acquired and analyzed by CPU 102, the result then being combined with the working images and provided as one or more output. Based on data analysis techniques other outputs, as described in more detail above, are also provided by CPU 102 assisting the User in performing the surgical procedure. The fluoro images are provided by the fluoroscopy system 118 and preferably comprise RAO and LAO views obtained from a bi-plane fluoroscope 110.

The preferred method of Fig. 9A is accomplished by a User, such as a cardiologist, or other surgeon performing a surgical procedure, or an assistant present during the surgical procedure aiding the cardiologist or surgeon, in combination with CPU 102 hardware under software or firmware control in combination with the fluoroscopy system 118 and video display system 106.

In an initial step 150, a video frame as part of the video signal 120 received from video source 114 by CPU 102 is digitized by the frame grabber. In a preferred embodiment, CPU 102 comprises two frame grabbers, each frame grabber digitizing a separate video frame from a separate fluoro video signal as part of video signal 120, the two separate video frames being digitized simultaneously, and each frame representing a different perspective or view, such as RAO and LAO views, respectively. The two digitized frames, since they are digitized simultaneously, are referenced to and, thus, represent the respective views at the same instance in time.

The digitized frames obtained in step 150 define working images which are used as a starting block for merging other data, as is described in more detail below. After the working images are defined, the working images are then registered with, or otherwise correlated with, various bodily signals produced by body systems of the patient, in a step 152. For example, ECG and respiratory cycle signals acquired by CPU 102, as described above, could be obtained by the data acquisition system when the frames are digitized in step 150, allowing the correlation between the current frame views and the acquired ECG and respiratory signals. In this regard, ECG and respiratory signals, along with other body system signals, are considered part of input instrument 108. Also contemplated as part of step 152 would be any required signal conditioning of the acquired bodily signals. Such conditioning may include filtering of electromagnetic or electrical noise as part of the signal acquired.

It should be apparent that the digitization of frames in step 150, rather than compared with existing bodily signals, could be performed in response to one or more bodily signals, such as an ECG signal, such that a digitized frame of substantially similar orientation is continuously acquired in step 150. For example, the RAO and LAO digitized frames could be acquired with respect to the end-diastole phase of the cardiac cycle resulting in substantially similar frames over a period of time. The frames could also be further correlated with a point within the respiratory cycle, such as the mid respiration point. As will later become more apparent, these measurements help to create a more accurate virtual depiction of the target tissue area of a heart generated as part of one or more CPU 102 outputs.

During the step 152, the digitized frames themselves may also be subjected to the same or different signal conditioning processes described above to eliminate undesirable noise or put the frame in a format more suitable for further analysis by CPU 102. Undesirable noise is meant to be defined broadly, when discussed in relation to digitized frames or other signals acquired, as the removal of any part of the digitized frame or acquired signal resulting in a clearer understanding of the information therein, whether determined from the User's point of view or the CPU 102 itself.

At the end of step 152 the CPU has acquired two fluoro video frames, defining two working images, and has correlated or registered these working images with various body system signals. The working images, along with other acquired data, while stored in random access memory of CPU 102, can also be stored on the non-volatile storage device of CPU 102 for later viewing or analysis. For example, after the procedure is performed, these working images and others stored could be reassembled into a histology of the procedure for review.

Once the digitized frames are registered in the step 152, CPU 102 enters a reactionary mode, reacting to one or more specific events occurring during the procedure. These events include, but are not limited to, a motion marker detection event, a V-gram detection event, an energy delivery device detection event, and a treatment event. Each of these events are provided to CPU 102 by the User's manipulation of one or more input devices 104. For example, with reference to Fig. 10E, the User may initiate an event by selecting a button B1 which may be labeled with the event, such as button "MOTION MARKER DETECTION" (specific labeling not shown). CPU 102 detects and interprets this input, in this case, as the motion marker detection event, in a step 160.

Alternatively, this or other events could be defined by the closure of a switch or switch combination, as part of input device 104, related to the specific event. Thus, the User can use the input device 104 to align a cursor or crosshair with a motion marker on the live fluoro. The User can then press a button defining the motion marker detection event and commanding the CPU 102 to interpret the current location of the crosshair as the location of the motion marker, as described in more detail below.

In order for the CPU 102 to correlate current and past working frames with live fluoro, a reference point defining the patient's body with respect to fluoroscope 110 must be defined. Motion markers are utilized by the combiner of the CPU to align the generated working frames with live fluoro views. Thus, when a patient's body rotates or otherwise moves with respect to a particular view, the working image can be rotated or otherwise oriented such that the working image is aligned with the respective live fluoro view. As described above, the motion markers can be radio opaque markers of various shapes and sizes strategically placed at known locations on or within the patient's body, remaining in place and within at least one perspective view, the RAO view for example, during the duration of the surgical procedure.

By way of example only, radio opaque markers '+' and '-' of Fig. 10A, and 'X' and 'O' of Fig. 10B are four types of such markers, the markers of Fig. 10A being placed on the chest region of the patient's body and the markers of Fig. 10B being placed on the left side of the patient's body.

Once the CPU 102 enters the motion marker detection mode it acquires the present location described by input device 104 in a step 162. Next, in a step 164, the orientation of the working image within the CPU 102 is manipulated such that the working image orientation is essentially similar to the orientation as observed in the live fluoro image. Therefore, if, for example, the patient's body rotates X° with respect to the center of the live fluoro image provided by fluoroscopy system 118 such that the location of motion marker "X" of Fig. 10A is shifted X° when compared to a working image overlaid onto the live fluoro as discussed below, the User could redefine a new motion marker location. In response to the new motion marker location, CPU 102 would rotate the working image X° in its memory prior to combining the working image with the live fluoro such that only one set of corresponding motion markers are visible in the output.

Once the motion registration markers have been obtained from the digitized frames, representative of the RAO and LAO views, the frames can then be correlated to previously obtained frames of the RAO and LAO views, respectively, by CPU 102. The placement of the motion registration markers may be made to allow the markers themselves to be fluoroscopically viewed in other planar views. Thus, for example, the side views of motion markers of the RAO view of Fig. 10A could be viewed in the LAO view as indicated by markers '+' and '-' of Fig. 10B. The additional views of these markers allows CPU 102 to more accurately correlate past working images with currently acquired frames or working images.

In a similar fashion to the motion marker detection event, CPU 102 checks for a V-gram detection event provided by the User in a step 170. In step 170 the CPU 102 looks for or otherwise detects whether a V-gram is presently being performed, i.e., a V-gram event has been provided by the User, as a prelude to obtaining outlined structural views, such as RAO and LAO views, of the left ventricle. If a V-gram is being performed, radio opaque dye is injected by the User into the left ventricle concurrently or immediately after the V-gram event. With the dye providing a contrast between the left ventricle chamber and the surrounding tissue, an outline of the left ventricle is performed in a step 172, as described in greater detail above. The outline images of the left ventricle obtained in step 172 are stored in either volatile or non-volatile storage devices in a step 174. Typically, the outline images are obtained once, at the beginning of the surgical procedure, and then are continually combined into the current working image in a latter step, discussed below. However, if required during the procedure, the User can obtain new outline images of the left ventricle per steps 170-174 as discussed above, the new images being stored or otherwise replacing the previously obtained outline images.

Additionally, the chamber of the left ventricle can be further defined by placing the distal tip of the energy delivery device against the wall of the heart and commanding the CPU 102 to acquire the location of the distal tip of energy delivery device, as is discussed in more detail below. The acquired points could then be stored and later utilized providing that location to the CPU 102

As with other events discussed above, CPU 102 checks for an energy delivery device detection event in a step 180. During a PMR procedure in accordance with the first embodiment of the present invention, the distal tip of the energy delivery device 300 is viewable on the live fluoro images by the User. As stated above, the device 300 may comprise sensors including radio opaque markers. The energy delivery device 300 of the first embodiment of the present invention comprises at least one radio opaque marker 312, the marker being placed at the distal tip 310 of the device 300 such that the distal tip is readily recognizable in the live fluoro. If, during the procedure, it is desirable to record or store the current position of the distal tip 310 of device 300, the User, using the input device 104, provides the position by placing the crosshair over the live fluoro image and commanding the CPU 102 to acquire the point defining the current distal tip 310 position in a first view, the RAO view for example, in a step 182.

Using a live fluoro freeze capability as part of the frame grabbers of the CPU 102, the User can acquire distal tip 310 distal tip position information in a second view, the LAO view for example, as part of step 182. The second view when combined with the first view provide information for the CPU 102 to generate a virtual two or three-dimensional view, or other desirable output described above. In a step 188, the position information obtained in step 182 is merged into the working image.

CPU 102 also checks for the occurrence of a treatment event in a step 200. The application of a desired therapy at a target tissue defines the treatment event. For example, activation of an energy source by depressing a foot switch, as an input device 104, could be used to define the treatment event. As stated above, the desired therapies include application of drugs or angiogenesis agents, other fluids, or ablation energies designed to remove or otherwise cause an injury to tissue. Once it is determined by CPU 102 that a treatment is in progress, the position of the distal tip 310 of energy delivery device 300 is obtained in a step 202 and combined with the working images in a step 208 in a similar fashion as in steps 182 and 188, respectively. More specifically, typically the treatment location is described in terms of the distal tip 310 of energy delivery device 300. Thus, the treatment location is the distal tip location obtained in step 182.

Prior to application of a desired therapy or treatment, sometimes it is desirable for the User to determine whether contact between the distal tip 310 of the energy delivery device 300 and the target tissue site has occurred. For example, this is desirable prior to activation of a laser energy source which results in the creation of a revascularization channel. Application of the laser energy source while the distal tip is not in contact with a wall of the left ventricle is undesirable since energy is applied directly to blood cells flowing through the ventricle rather than wall tissue itself.

As a preliminary step 200A of step 200, therefore, the User would not enable an energy source, providing CPU 102 with a treatment event, until the distal tip 310 of the device 300 is in contact with a wall surface of the left ventricle. Contact between the distal tip 310 and the wall surface can be estimated from a comparison of the distal tip 310, as viewed in the live fluoro image, with the outlines of the left ventricle made part of an output of CPU 102 as discussed herein. A confirmation of heart wall contact is made when the User observes the distal tip 310 of device 300 moving synchronously with the beating of the heart and, more specifically, the contraction and relaxation of the left ventricle itself. At this point the User can pass the treatment event to CPU 102 resulting in the acquisition and manipulation of data as discussed above with regard to steps 202 and 208. Wall contact may or may not be a prerequisite to the application of the desired therapy.

In a step 210 the working images, including the V-gram outline images and other data acquired by CPU 102 as described herein, are combined with respective live fluoro views, the combined output being provided by CPU 102 to video displays 116, and video display system 106 as an output video signal 122 for viewing by operating room personnel. Therefore, the fluoro video 120, comprising video signals representing RAO and LAO views, received by CPU 102 is combined with the saved outline images of the corresponding perspective view by the combiner and provided as the video signal 122 at the video output port. Therefore, the video display 116 could contain, for example, the live fluoro view from the RAO perspective with the RAO-O outlined image as shown in Fig. 2B superimposed thereon giving the cardiologist an estimation of the left ventricle shape along its longitudinal axis. Similarly, a view corresponding to the LAO perspective with the LAO-O outlined image as shown in Fig. 2C is provided by CPU 102 to video display 116.

Additionally, as described above, other outputs can be generated by CPU 102 in a final step 220 to be displayed on one or more video displays 116, 116a and/or an additional display as part of the video display system 106, in response to frame data and other data acquired in accordance with the present invention and at the request of the User.

Steps 150 through 220 define a main loop which is to be performed frequently enough to allow for useful visual response, typically between 3 and 10 Hz. Also, the User inputs resulting in various events being provided to CPU 102, rather than being performed sequentially as part of the method of Fig. 9A, could be interrupt-based events. That is, the main loop could be simply defined as steps 150, 152, 210, and 220, and User inputs defining the events discussed above would interrupt the processing of main loop steps and force CPU 102 to momentarily address and process the current event provided.

For example, with the distal tip 310 against a target tissue site, the User, using the input device 104, would provide to CPU 102 an input defining a treatment event. The CPU 102 would stop running one of the main loop steps 150, 152, 210, or 220, and execute steps 202 and 208. The CPU 102 would then return to and continue executing main loop steps after execution of step 208. Such a system is more efficient since it forces the CPU to focus on tasks desired by the User. The gains of such a system are more realized below in discussing further embodiments.

With reference also to Fig. 10, the combination of software executed by CPU 102, manual input provided by the User, and selected outputs generated by CPU 102 in accordance with the first embodiment of the present invention can be better understood. The User, through a software interface of CPU 102, such as a button B2, as shown in Fig. 10E, which may be labeled "DEFINE TREATMENT LOCATION" (specific labeling not shown), notifies and commands the CPU 102 to acquire a data point ($XI_{RAO}$, $YI_{RAO}$) corresponding to the distal tip 310 of an energy delivery device 300 (not shown) in a first view, the RAO view for example. The point is defined, as described above, by moving a crosshair over the distal tip 310 and then signaling the CPU 102, by clicking the mouse for example, to acquire the data point in the RAO view. In a similar fashion, a data point ($XI_{LAO}$, $YI_{LAO}$) is acquired with respect to a second view, the LAO view for example, as shown in Fig. 10B.

With two data points entered or otherwise provided to CPU 102 by the User, a two or three-dimensional depiction of each data point with respect to other data points or structures can be created as part of one or more system output described above.

These two, two-dimensional data points obtained with respect to two different views, RAO and LAO views for this example, along with the angular relationship between the two views can be combined to generate a three-dimensional data point. The generated three-dimensional data point is defined by two lines, a first line perpendicular to the first view and a second line perpendicular to the second view, each line passing through or intersecting a two-dimensional data point of interest, the distal tip 300 of energy delivery device 300 for example. The three-dimensional location is then calculated to be the midpoint of the shortest line segment between these two lines, if any. Note that these calculations do not require the two views to be orthogonal, but rather that they not be coincident.

For example, the outlined images of the left ventricle, since the orientation planes are known, can be represented in a two-dimensional depiction as shown in Fig. 10C or a three-dimensional depiction as shown in Fig. 10D. Further, a first generated data point ($XI, YI, ZI$), since its orientation with respect to the RAO and LAO outlines is known, can be merged into the same three-dimensional depiction, as shown in Fig. 10E and described in more detail above. During the surgical procedure, a number of additional treatment location points n, $(X2,Y2,Z2)$ ... $(Xn,Yn,Zn)$, are later obtained and merged with the RAO and LAO outline views and other data as part of current working images, and later combined with the live fluoro by CPU 102 for display on video screens 116, 116a, and/or a video display as part of video display system 106.

In a more preferred embodiment, the catheter location data points obtained in step 182 are stored in non-volatile memory such that each data point or a defined group can be recalled and merged into the current working image in step 188, at the request of the User. For example, the User can define a series of data points as a first row of treatment locations. The row can then be merged, at the request of the User, into any given working image and combined with the live fluoro image for viewing in one or more outputs of CPU 102 as defined above. This allows the User to better interpret and differentiate one set of channels, for example, on one interior chamber wall of the left ventricle from another set. This also allows the User to systematically display various groups of channels to better understand the position and orientation of such channels with respect to other sets or groups of channels.

As stated above, the steps of Fig. 9A define a program loop continuously executed within predetermined loop-cycle time limits. In a more preferred first embodiment of the present invention, inputs other than V-gram, and treatment related events directly provided by the User, would not be required. Rather, the CPU 102 would be incorporated with additional software comprising one or more algorithms directed to performing object detection, motion detection, edge-detection, or other image-analysis to, for example, automatically determine the location of various radio opaque markers. Thus, in the step 160 of Fig. 9A, the CPU 102 would analyze the current digitized frames obtained in step 150 to determine the location of the motion registration markers. Characteristics specific to individual markers help to differentiate the markers during CPU 102 analysis.

The CPU 102 would then analyze the frame and compare image portions in high contrast to surrounding portions with the known images of the motion registration markers utilized during the surgical procedure. Once the location of the registration markers is determined, the CPU 102 would combine this information into the working frames, as discussed above.

Additionally, in step 170, the ventricular outline following a contrast injection in fluoroscopic acquisition of a left V-gram can be acquired using edge-detection algorithms. Thus, as part of the step 170, the CPU 102 would analyze a digitized frame after the radio opaque dye has been injected into the left ventricle of the heart, based on a V-gram event provided by the User. Applying edge-detection algorithms to the contrast produced by the dye in the acquired frame, CPU 102 will determine the left ventricle structure and produce the outlined RAO and LAO views as discussed above.

Using an edge-detection algorithm similar to the one used in step 170 immediately above, the distal tip 310 of an energy delivery device 300 having a distal tip 310 radio opaque marker 312 attached thereto can be detected in the digitized frame in step 180. Furthermore, the obtained position of the detected distal tip 310, during analysis of several consecutive frames, can be compared with an ECG signal supplied to CPU 102 to determine whether the distal tip 310 is in contact with the left ventricle wall, as part of the wall contact preliminary step 200a.

In similar fashion, treatment areas acquired in the step 202 are detected and combined into the working images through the use of frame analysis incorporating detection algorithms by CPU 102, as discussed above regarding steps 200-208. Additionally, when the CPU 102 detects a Treatment event defined above, rather than acquiring a point defining the application of a desired therapy, the CPU 102 could acquire a treatment area defined by the specific acquisition of a number of points further defined by the movement of the distal tip of energy delivery device 300 during the treatment event. The acquired treatment area would then be merged into the current working image in the step 208.

If wall contact is a prerequisite to application of a given therapy the energy delivery device 300 could comprise a functional device which would then detect wall contact and provide the information to the CPU 102 for interpretation and control purposes. For example, the functional device could comprise a pressure transducer which is then used to detect wall contact as described in EPO Publication No. EP 0 868 923 A2, entitled "Steerable Catheter with Tip Alignment and Surface Contact Detector", published on March 31, 1999.

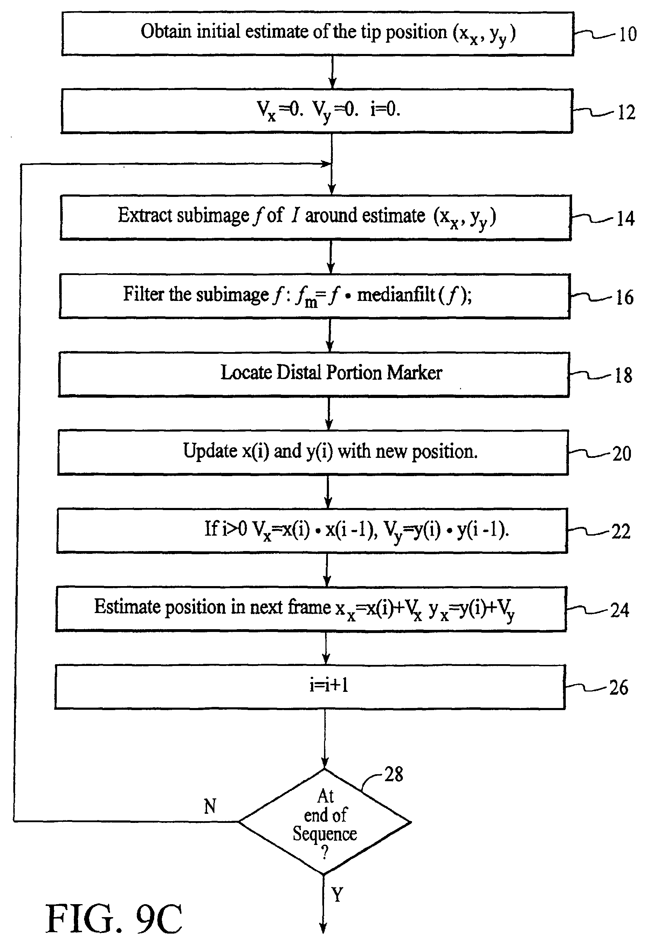

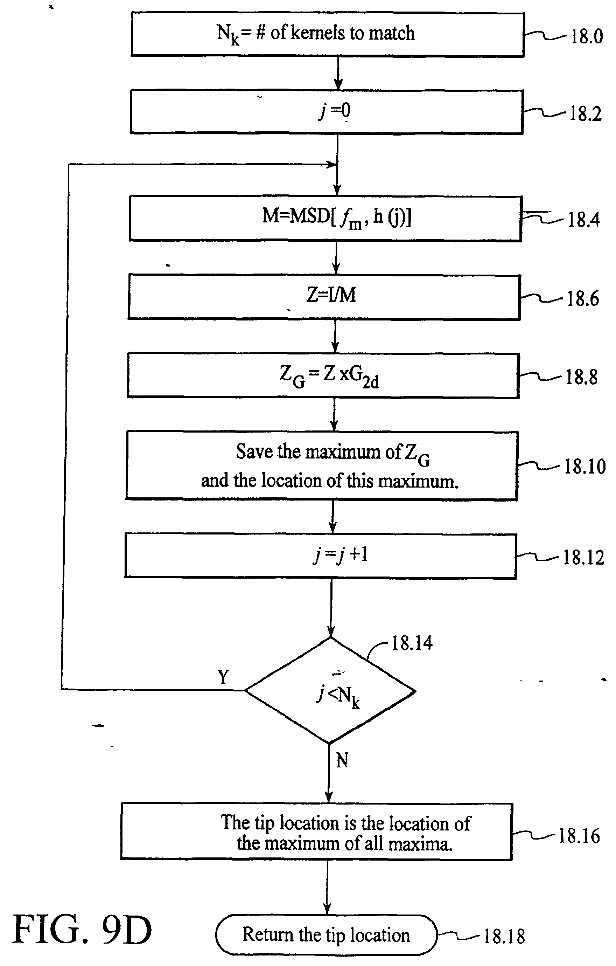

Now with reference to Figs. 9C and 9D, a preferred object, motion, and edge-detection algorithm for the detection of radio opaque markers on an energy delivery device 300 as part of step 182 used during a surgical procedure in accordance with the first embodiment of the present invention can be more readily understood. The algorithms defined below don't detect the distal tip 310 itself. Rather, the algorithms detect the radio opaque marker 312 as part of the distal tip 310, providing a relative location of the distal tip 310. Additionally, as the algorithms defined below can detect the marker 312 on the distal tip 310, the algorithms can also detect other markers placed along the energy delivery device, as discussed in more detail below.

Figs. 9C and 9D define a main algorithm and a sub algorithm, respectively, which provide CPU 102 catheter tip location and orientation information. The algorithms are based on User supplied parameters which can be provided to the CPU 102 in any suitable matter, such as with a keyboard or other alphanumeric data entry device. Algorithm parameters include the size of an extracted sub-image as part of the working image to analyze; the extent to which the sub-image data is filtered; the number of representative images to match with the sub-image; and the variance parameter for a Gaussian function.

With particular reference to Fig. 9C, an initial estimate is obtained by CPU 102 of the distal tip 312 position in an initial step 10. The estimation provides a starting point for the CPU 102 algorithms defined by Figs. 9C and 9D to initiate detection of the marker 312 on distal tip 310. It also allows for faster tip 312 recognition, reducing the area CPU 102 must investigate in its search for the exact location of tip 312, as will become more apparent below.

The estimation of tip 310 position is provided to CPU 102 by the User using the input device 104 and techniques discussed above with regard to marking data points. Alternatively, the CPU 102, during step 10, could initiate a systematic search for the tip 312, as part of a step 10A (not shown), by analysis of the working images obtained from the digitized frames as described above. As is described in greater detail below, the algorithm of Fig. 9C can be used to search the entire area of a given working image. The CPU 102, in examining the entire image, may find several prospective tip candidate points, one true tip and other erroneous tips. The CPU 102 would then analyze each candidate using algorithms described herein and related to Figs. 9C and 9D to find the one true distal tip marker 312.

After the initial estimate of tip 312 position is obtained by CPU 102, a variable initialization step 12 is performed. During step 12, variable i, representing the number of working images considered during the current sequence, is initialized to equal zero and tip 312 velocities in the *x* and *y* direction of the working image, i.e., $V_x$ and $V_y$, are initialized to zero. Here, the variables *x* and *y* are variables representing any two-dimensional space. Thus, if the working image is the RAO view the dimension variables would be *x* and *z*, rather than *x* and *y*.

As shown, a step 14 defines the start of a main loop of the algorithm of Fig. 9C. During step 14 a small sub-image *f* surrounding the current location of distal tip 312 is extracted from the current working image. The sub-image of a predetermined size defined by the User is selected and obtained from the working image to reduce the possibility of erroneous detection of tip 312 and speeds the processing of the algorithm of Fig. 9C since it dramatically reduces the area which CPU 102 must analyze. The selected size can be in any suitable form including video elements, such as pixels, or memory characteristics, such as bits, bytes, words, etc. Since this is the first time step 14 is executed during the current sequence the current location used would be the estimation obtained in step 10. However, during analysis of consecutive working images as part of the current sequence, the current location would be the actual location calculated in a previous iteration of the main loop.

The sub-image *f* is then filtered in a step 16 by subtracting a median-filtered version of the sub-image *f* from itself, the step being represented by the mathematical expression $f_m = f$ - median-filtered(*f*), as shown. The filtering of step 16 removes slowly varying brightness changes from the sub-image $f_m$ and thus causes the background detail of the working image, as part of a digitized fluoro image, to contrast against the moving radio opaque tip marker 312 of energy delivery device 300. The distal tip marker 312 of the energy delivery device 300 is then located, due to analysis of contrasting elements within the filtered image $f_m$, in a step 18.

The newly acquired catheter tip marker 312 location is then stored by CPU 102 in memory as *x* and *y* coordinates with respect to the current working image *i*. Therefore, for working image 0 (*i* = 0), the point (*x(0),y(0)*) would represent the currently acquired coordinate for the distal tip marker 312.

In a step 22, velocity vectors $V_x$ and $V_y$ are calculated by subtracting the last marker 312 coordinate position from the currently obtained position for a working frame > 0 (*i* > 0) as part of the current sequence. A distal tip 312 location estimation is then calculated in a step 24 by summing the currently obtained tip 312 position with the effects of the tip velocities calculated in step 22. The tip location estimation, coupled with other advantages noted above with regard to the algorithm of Fig. 9C, improve the efficiency of energy delivery device distal tip detection.

The time required for the execution of software or firmware performing the individual steps of the algorithm of Fig. 9C can be ascertained, each step requiring a specific number of CPU cycles defining a finite time period for execution. Therefore, while the velocity vectors of distal tip 312 are shown being added to point coordinates, in fact, these velocities and coordinates are related to a time element to allow for such calculations.

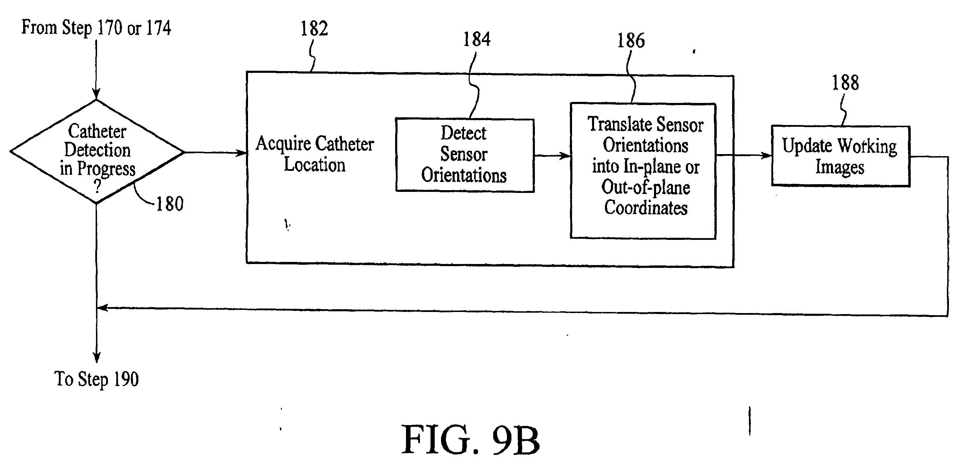

After the estimation is made in step 24, the variable *i* is incremented in a step 26 and an end of sequence determination is then made in a step 28. If all working images of a sequence (*i*-1) equals the predetermined value, the main loop defined by steps 14 through 28 is exited and the step 182 of Fig. 9B is deemed completed. If other working images are left to be analyzed, algorithm execution continues at step 14 and advances as discussed above until the end of sequence determination in step 28 is true.

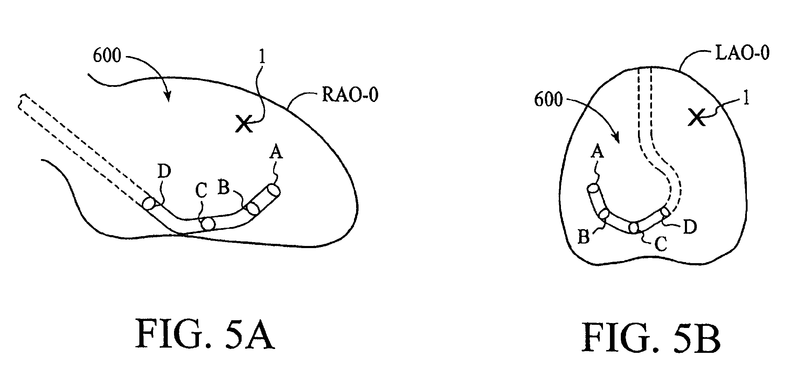

With reference to Figs. 5A and 5B, a second embodiment of an enhanced tracking system in accordance with the present invention, incorporating features described above pertaining to the general embodiment, can be more readily understood. The second embodiment of the present invention differs from the first in that the energy delivery device comprises multiple radio opaque markers on or near its distal portion. All or several of the radio opaque markers A-D are similar in size and shape and are placed a known distance from each other along the distal portion of an energy delivery device, as shown in Figs. 5A and 5B.

Radio opaque markers A-D can be of any form discussed above with reference to marker 412, 512 on the distal tip 412, 510 of catheter 400, 500. Energy delivery device 600 can be catheter 400, catheter 500, or any other energy delivery device discussed herein incorporating a plurality of markers. As with the first embodiment, in this second embodiment fluoroscopy system 118 also acts as part of input instrument 108, providing, in part, position and orientation information regarding the energy delivery device.

Since the live fluoro image would only show markers A-D, seemingly floating in space, it is desirable to represent the position and orientation of the distal end of the energy delivery device 300 in a way that is easier to interpret by the User during a surgical procedure.

Now with reference also to Fig. 9A, a procedure in accordance with the second embodiment of the present invention will be discussed. Figs. 5A and 5B depict an energy delivery device 600 within the left ventricle of the heart, the outlines RAO-O and LAO-O, obtained as discussed above with reference to steps 170-174, and defining the left ventricle structure in a similar manner as described above. As with the marker on the distal tip 410, 510, markers A-D are detected in steps 180-182 and combined with the working image in step 188 for display using techniques described above. Further, in the step 188, CPU 102, using additional interpolation algorithms, connects markers A-D resulting in a more descriptive representation of the position and orientation of the distal portion of energy delivery device 600.

The CPU 102 calculating and generating the catheter orientation depiction can provide the resulting representation in any suitable form. For example, depending on the performance characteristics of the CPU 102, the distal portion of the energy delivery device 600 may be represented by a line which starts at the center of detected marker A and ends at the center of marker D, passing through the centers of markers B and C. Alternatively, the distal portion of the device may be represented by two lines essentially parallel and running tangentially along the detected surfaces of markers A-D, as shown in Figs. 5A and 5B. The portion of the energy delivery device 310 in dashed is representative of the portion where the specific orientation may be unknown. Additional markers placed along the dashed portion of the device would enable that portion, as well as the more distal portion, to be detected and represented in an overlaid working image by CPU 102.

Therefore, as a result of the preferred method of Fig. 9A, while the live fluoro provides viewing of the markers themselves, the CPU 102, using algorithms discussed above, generates a position and orientation depiction of the catheter 600 and combines this depiction, as part of the working images, with live fluoro resulting in views as depicted in Figs. 5A and 5B. Such views aid the User, and specifically the cardiologist or surgeon, in the placement of catheter 600 having a specific orientation within a confined area, enabling the distal tip 610, for example, to apply requisite force to target tissue during the application of a desired therapy.

CPU 102, as is discussed above relative to the first embodiment, can incorporate certain User tasks such as the manual identification of the markers B-D along the catheter 600 proximal from the distal tip 610 along with the distal tip 610 marker A. Such markers A-D, for example, can be automatically obtained by the CPU 102 and dynamically displayed as discussed above. Additionally, during a treatment event the distal marker A location can be acquired and stored as described above.

The algorithm of Fig. 9C can be used to locate all markers A-D of the second embodiment of the energy delivery device, when the algorithm is used to process working images from both, RAO and LAO, views. Such analysis results in the fast and accurate position and orientation depiction of energy delivery device 600 within the working images and, ultimately within the live fluoro, as described above.

Additionally, it should be apparent from the foregoing that the LAO view itself can be completely generated by CPU 102 and include depictions of the distal portion of the energy delivery device, including markers A-D, and, optionally, the LAO outline view, when one or more of the markers A-D are asymmetric, as discussed in more detail below with regard to additional embodiments incorporating different sensor arrangements. Asymmetric markers allow for the more accurate interpretation of certain ambiguities observed when the distal tip of the energy delivery device is directed in an in-plane or out-of-plane direction with reference to a plane of view. Thus, the User could view one or more video displays 116, 116a comprising a live fluoro RAO view combined with the corresponding RAO view working image and the CPU 102-generated LAO view.

With reference to Figs. 4B, 4C, and 4D, a third embodiment of an enhanced tracking system in accordance with the present invention, incorporating features described above pertaining to the general embodiment, can be more readily understood. The third embodiment of the present invention differs from the first and second embodiments in that the energy delivery device comprises multiple radio opaque markers specifically designed to allow position and orientation information to be acquired by CPU 102 through the analysis of a single view, preferably the RAO view, received from the fluoroscopy system 118.

With specific reference to Fig. 4B, a distal tip 510 radio opaque marker 520 as used in accordance with the third embodiment of the present invention can be more readily understood. More specifically, Fig. 4B depicts representative views of the marker 520 as perceived by a fluoroscopy system such as system 118.

Marker 520 is a thin-walled cylindrical marker made from similar materials as other markers described herein. As shown, depending on the orientation of marker 520, the marker 520 appears having distinct shapes. Marker 520A is a representative fluoro view of marker 520 where the central axis of marker 520 is perpendicular or normal to the fluoro plane. Thus, only the circular appearance of the thin wall of marker 520 is visible, providing an indication that the distal tip 310 of an energy delivery device 300 is either directed toward the X-ray source, hereinafter described as an out-of-plane view, or away from the X-ray source, hereinafter described as an in-plane view.

Marker 520D is shown oriented such that its central axis is parallel to the fluoro plane producing an essentially rectangular image of predetermined dimensions in the live fluoro, providing an indication that the distal tip 310 of an energy delivery device 300 is oriented parallel to the fluoro plane view. Markers 520B and 520C are representative views of marker 520 at axis orientation angles between those of markers 520A and 520D, 0° and 90° respectively.

As the marker 520 is rotated about axis line A, its shape changes from a circular shape, as shown by marker 520A, to an elliptical shape having various dimensions, as shown by exemplary markers 520B and 520C, to a rectangular shape, as shown by marker 520D. Each of these shapes are distinct and, therefore, are readily and separately perceivable in a live fluoro video image.

Now turning also to Figs. 4C and 4D, utilization of marker 520 with a catheter 500A will be better understood. As shown in Figs. 4C and 4D, an RAO view is depicted with the RAO outline image RAO-O representing the left ventricle. As discussed above, the RAO-O image was generated earlier prior to the procedure and incorporated or combined with a live fluoro image depicting portions of a catheter.

Catheter 500A is an exemplary catheter shown incorporating the marker 520 of Fig. 4B. Unlike the catheter 500 of Fig. 4A, catheter 500A of Figs. 4C and 4D comprises a laser catheter 504A different from the laser catheter 504 of Fig. 4A, as is described in more detail below. The aligning catheter 502 may have one or more segments S defined by an angle formed therebetween, as shown. The laser catheter 504A, however, contains or includes a sufficient amount of radio opaque material to allow laser catheter 504A to be only partially visible in live fluoro when the catheter 504A is parallel to the fluoro view.

For example, the radio opaque marker 520 is made of a material which absorbs or otherwise blocks the transmission of X-rays and thus appears in live fluoro as a dark area equivalent to the objects shadow produced on a surface when the object is held in sunlight. In contrast, laser catheter 504A contains a radio opaque substance, such as a radio opaque powder, within its main wall surface. As the orientation of the laser catheter changes, the image produced by the catheter 504A in live fluoro will darken or produce a change in the degree of opacification since, at times, more radio opaque material lies between the X-ray source and the image detector of fluoroscopy system 118.

Additionally, laser catheter 504A may incorporate one or more symmetric or asymmetric markers 514 (not shown for clarity) along its distal portion at known locations and orientations with respect to each other, as well as one or more longitudinal markers 518 defining essentially planar surfaces and lying parallel to the plane of deflection of laser catheter 504A, to help CPU 102 better determine the position and orientation of the distal portion of catheter 500A, as will be discussed in more detail below. Similarly, aligning catheter 502 may include one or more symmetric or asymmetrical markers 516 along its distal portion, at known positions with respect to each other. For example, a first asymmetrical marker 516A may be placed on a segment $S_1$, while a second asymmetrical marker 516B is placed on a segment $S_2$, markers 516A and 516B defining an angle and positional orientation therebetween.

An example of positional orientation is found where the segment $S_1$ is placed on a first surface of the catheter 502 while the segment $S_2$ is placed on an opposing surface. Alternatively, a marker 516C may be placed on aligning catheter 502 extending along two or more sections S providing another known orientation from which CPU 102 can analyze. These known marker placement positions and orientations, as well as other components of catheter 500A comprising variable amounts of radio opaque material resulting in differing degrees of opacification based on orientation, as stated above, aid the CPU 102 in generating out-of-plane images as is more fully discussed below.

Also shown in Figs. 4C and 4D are exemplary views of the clock face output. As discussed above, the clock face graphical output generated by CPU 102 provides the User with an indication of catheter 500A distal tip 510 orientation with respect to a different view, an LAO view for example. Thus, referring specifically to Fig. 4C, the catheter 500A is shown with the distal tip 510 pointed toward the inferior surface of the left ventricle as depicted by RAO-O. Marker 520 is shown with the rectangular shape of marker 520D providing an indication that the distal tip 510 lies parallel to the fluoro plane, i.e., is not rotated in-plane or out-of-plane. The corresponding view from the LAO perspective would show the distal tip 510 forming a 0° angle with the fluoro plane and thus be pointing towards the inferior surface of the left ventricle.

In contrast, referring specifically to Fig. 4D, catheter 500A is shown with the laser catheter 504A rotated such that the distal tip 510 is pointing either in-plane or out-of-plane with respect to the fluoro plane. As will be discussed in greater detail below, without analysis of the catheter components, including for example, the marker 520 having an orientation similar to marker 520D, it is hard to distinguish which direction the catheter tip 510 is pointing, in-plane vs. out-of-plane. Here, the distal tip 510 is pointing in-plane and thus the clock face image, which generally depicts the LAO view, indicates the distal tip 510 pointing more to the lateral wall of the heart.

Given the structure of catheter 500A and with reference also to Figs 9A-D, a preferred procedure involving the third embodiment of the present invention can be better understood. As stated above, it is desirable to calculate the position and orientation of energy delivery devices within a body during a surgical procedure incorporating a fluoroscopy system 118 having a single-plane fluoroscope and where it is undesirable to rotate the fluoroscope to obtain a second view, an LAO view for example, reducing the time of the procedure and exposure to X-ray radiation during the procedure. The preferred method involving the third embodiment of the present invention builds upon or otherwise adds to the preferred method of Fig. 9A.

When only a single-plane fluoroscope 110 is utilized producing a single view, preferably an RAO view, the distal tip orientation is analyzed and provides position and orientation information with respect to another view, an LAO view for example. With reference to Fig. 9B, the distal tip marker 312 orientation must be detected in a step 184 as part of step 182. Once the orientation of the marker 312 has been determined, a translation of the orientation information into in-plane or out-of-plane coordinates is performed in a step 186, also part of step 182. The orientation of the marker 312 is determined by the sub-algorithm of Fig. 9D, the sub-algorithm being a further definition of step 18 of the main algorithm of Fig. 9C. It is important to note that with other input instruments 108 being utilized a portion or all of the data corresponding to data acquired during execution of steps 184 and 186 may be provided to the data acquisition system of CPU 102. For example, a specific input instrument 108 may provide sensor orientations but not provide these orientations with reference to other data acquired by CPU 102. The CPU 102 would then correlate the sensor data to the other data acquired, providing the coordinates per step 186.

Now with specific reference to Figs. 9C and 9D, the steps which make up a distal portion marker location algorithm incorporating tip orientation information, depicted as step 18 of Fig. 9C, can be more fully understood. The distal portion marker location algorithm generally functions by comparing one or more stored images of the distal tip 310 of the catheter 300 to the current sub-image $f_m$ as part of the current working image. The stored subimages are termed kernels, each kernel representing the catheter tip 310 having a radio opaque marker 312 in a specific orientation.

In a first step 18.0 a variable $N_k$ is set to the number of kernels which will be considered during execution of the distal portion marker location sub-algorithm. The greater the number of kernels provided results in a more accurate determination of the marker orientation. In a second step the kernel counter $j$, defining the current kernel being considered, is initialized to zero.

In a step 18.4, the first step of a main loop of the sub-algorithm, a Mean-Squared-Difference (MSD) matching filter is used to compare the sub-image $f_m$ of step 16 with the current kernel, defined as $h(j)$. The MSD filter itself is defined as:

$$M(a,b) = \frac{1}{KL} \sum_{k=-K/2}^{K/2} \sum_{l=-L/2}^{L/2} [f_m(a+k, b+l) - h(k,l)]^2$$

Where:

$h(k,l)$ = Value of the current kernel at pixel location $(k,l)$;
L = Length of the kernel in pixels; and
K = Width of the kernel in pixels.

The output of the MSD filter produces a small value when the sub-image $f_m$ and the current kernel, of the stored kernels, is found to be very similar. With increasing similarity between the current kernel and the sub-image $f_m$, the MDS output approaches a value of zero.

The image Z is then set to the reciprocal of the MSD output in a step 18.6. The image Z, therefore, is maximized when the sub-image of the working image and the current kernel are nearly identical. In a step 18.8, the image Z is then multiplied by a two-dimensional Gaussian function, $G_{2d}$, that obtains its maximum at the center of $Z_G$, the expected catheter tip 310 location. Multiplying the image Z by $G_{2d}$, in the step 18.8, gives more weight to peaks that are close to the expected catheter tip 310 location.

The value and location of the maximum of $Z_G$ are found and stored in a step 18.10. In a step 18.12 the kernel counter variable, $j$, is incremented. Next, if the kernel counter variable is found to be less than the number of stored kernels, i.e., all the kernels have not been compared with the current sub-image, transfer is passed back to step 18.4, in a step 18.14, and execution of the sub-algorithm continues as discussed above.

If all the stored kernels have been considered then the tip 310 location is then determined in a step 18.16. The catheter tip 310 is located at the point corresponding to the maximum of $Z_G$ of all the maxima. The coordinates, associated with pixel coordinates $(k,l)$ of the kernel which most closely matches the sub-image, as well as orientation information, i.e., the orientation of the kernel which most closely matches the sub-image, is provided to step 20 of the main algorithm of Fig. 9C in a final step 18.18.

Therefore, by analyzing the specific shape of the distal tip marker 312, CPU 102 can estimate the position and orientation of the distal tip 310 of the energy delivery device 300 and provide one or more outputs, such as the clock face depiction, as discussed above. The CPU 102 can discern in-plane rotational movement from out-of-plane movement by referencing rotational motion of the laser catheter, the aligning catheter, and translational motion of the fiber optic or fiber bundle 508 with respect to the distal tip 310. The rotational movement of the catheters 502, 504A and the translational motion of the fiber optic 508 can be measured in any convenient manner, for example through the use of rotation encoder systems or potentiometers. Signals representative of signals provided by these measurement devices are then provided to the data acquisition system of CPU 102 for acquisition, interpretation and integration into one or more working image in accordance with embodiments of the present invention. Optionally, in-plane and out-of-plane motion can be distinguished by the User providing an additional input to CPU 102, such as a keyboard input "L" for describing the distal tip 310 directed laterally and input "S" for describing the distal tip 310 directed septally.

With respect to the catheter 500A of Figs. 4C and 4D, the algorithms of Figs. 9A-D may be utilized in a similar fashion in order to interpret orientations of markers 516 and 518 on the aligning catheter and laser catheter, respectively, allowing the CPU 102 to more accurately define output depictions. For example, starting from a known orientation such as the orientation depicted in Fig. 4C, the CPU 102 in accordance with methods described herein can associate movements of the marker 518, which is placed on side wall of the laser catheter. to indicate rotation of the laser catheter 504A. Additionally, since the curvature of the laser catheter 504A is known or predetermined, the degree of foreshortening of the distal portion of the laser catheter 504A, represented by X and Y measurements, of the distal portion of the laser catheter 504A observed will then define an angle of rotation. The X and Y measurements can be acquired by the CPU 102 in accordance with the present invention and utilized in the generation of one or more outputs, such as the clock face outputs as shown in Figs. 4C and 4D, and discussed above.

Additionally, the amount of rotation of the distal tip of laser catheter 504A can be enhanced by embedding a radio opaque filler material, such as barium sulfate or bismuth subcarbonate, typically between 10% and 40% loaded by weight, into the distal tip such that an image of varying opacity will be observed in the live fluoro when the distal tip is rotated from an in-plane position to an out-of-plane position with respect to an RAO view. The minimum opacification being perceived when the distal tip of the catheter 504A is directed parallel to the RAO view and the maximum opacification being perceived when the distal tip of the catheter 504A is directed either in-plane or out-of-plane with respect to the RAO view.

The analysis of markers 516 and 518 may be made in addition to acquisition of rotational and translational movements of the catheters 502, 504A and fiber optic 508, respectively, to more accurately define the current position and orientation of the catheter 500A.

With reference to Figs. 4E-5H, a fourth embodiment of an enhanced tracking system in accordance with the present invention, incorporating features described above pertaining to the general embodiment, can be more readily understood. The fourth embodiment of the present invention differs from the third embodiment in that the energy delivery device comprises multiple radio opaque markers of similar size and shape and arranged on the energy delivery device a known distance between each marker specifically designed to allow position and orientation information to be acquired by CPU 102 through the analysis of a single view, preferably the RAO view, received from the fluoroscopy system 118. This embodiment is particularly useful when a tip-deflectable energy delivery device, such as catheter 400, is utilized during a surgical procedure.

It should be apparent that while the markers are shown to be equidistant from each other, the markers only need be placed defining a known relationship between each marker. Additionally, the markers are shown to be of similar size and shape, but may also be implemented such that one or more markers are asymmetric to assist CPU 102 in more accurately resolving certain in-plane and out-of-plane ambiguities. With specific reference to Fig. 4E, the catheter 400 is shown having multiple thin-walled markers 414 on a distal portion 416. As is better shown in Fig. 4G, as the catheter 400 distal portion 416 is deflected by the deflection mechanism such that the distal tip 410 moves either out-of-plane or in-plane with reference to a specific view, the RAO view for example, the markers 414 indicate the movement in a similar fashion as marker 520, the distal portion 416 having the greatest deflection is shown producing a fluoro image closer to a circle. Since the markers 414 closer to the main shaft 402 appear as thin rectangular portions and the markers 414 closer to the tip 410 appear having an ellipticity, the distal tip 410 must be deflected either in-plane or out-of-plane from the main shaft 402.

In contrast, as shown in Fig. 4F, when the deflection of the distal portion 416 is essentially along the plane of view only the most distal marker 414 indicates a slight ellipticity. Therefore, the deflected distal tip 410 of catheter 400 is deflected in a plane roughly parallel to the plane of view.

Since the markers of catheter 400 are part of the deflectable distal tip portion 416, while the distal portion 416 deflects the wall portions of the distal portion 416 define an inner and outer bend radius along the plane of deflection. Therefore, the spacing between the markers 414 changes when the distal portion is deflected, the portions of the markers 414 essentially lying along the outer bend radius being a further distance apart than the portions of the markers 414 essentially lying along the inner bend radius. For example, with reference to Fig. 4G, a point A of a first marker 414 is shown as part of the outer bend radius of deflection and a point C is shown as part of the inner bend radius of deflection. Additionally, a second marker 414 comprises a point B and a point D as part of the outer bend and inner bend radius of deflection, respectively. Therefore, when the distal portion 416 of catheter 400 is deflected as shown, the distance between the points A and B is greater than the distance between points C and D providing the conclusion that the distal portion 416 is deflected essentially in-plane.

As discussed in greater detail above with reference to Figs. 9A-D, the algorithms of Figs. 9A-D can be used to determine the position and orientation of the distal portion 416 of catheter 400 by specifically determining the orientation of distal tip 410 marker 412, and then analyzing the circumferential spatial relationship of the markers 414 as between each other, the largest distances observed between different markers 414 defining the outer bend radius of deflection and, thus, defining the orientation of the distal portion 416.

Now with reference to Fig. 4H an alternative catheter 400A is shown. The catheter 400A of Fig. 4H, like the catheters 400 of Figs. 4E-4G, is adapted to have multiple markers 414A arranged in a known relationship to each other along the distal portion 416. However, the markers 414A, as shown, are similar in size and shape to marker 412. Therefore, only one marker need be defined by the User prior to execution of the algorithms of Figs. 9A-D and the algorithms of Figs. 9A-D determining the orientations of each marker 414A, without regard to bend radius, each marker 414A then specifically defining the position and orientation of the energy delivery device at its location along the distal portion 41.

Optionally, catheter 400A can comprise another radio opaque marker 414B, essentially rectangular in shape, acting as a bridge between the two most proximal markers 414A, as shown in Fig. 4H. This "I-beam" marker 414A/414B configuration, which is defined in more detail in the Rosenthal reference, provides additional information from which CPU 102 can calculate in-plane and out-of-plane projects of the distal portion 416 of the energy delivery device.

More specifically, marker 414B is placed on the catheter 400A essentially parallel to the plane of deflection of the catheter 400A. Thus, when the catheter distal tip 410 is directed in-plane, the marker 414B will appear as a thin line along a first side portion of the catheter distal portion 416 between the two most proximal markers 414A. In contrast, when the distal tip 410 is directed out-of-plane, the marker 414B will appear as a thin line along a second side portion appearing in opposition to the first side portion.

Therefore, the CPU 102, analyzing a current working frame as part of methods in accordance with the present invention, can utilize the marker 414B to better define and describe the position of the distal portion 416, including the distal tip 410, when combined with a live fluoro view, generating the combined image as one of several outputs based on a single planar view, such as an RAO view.

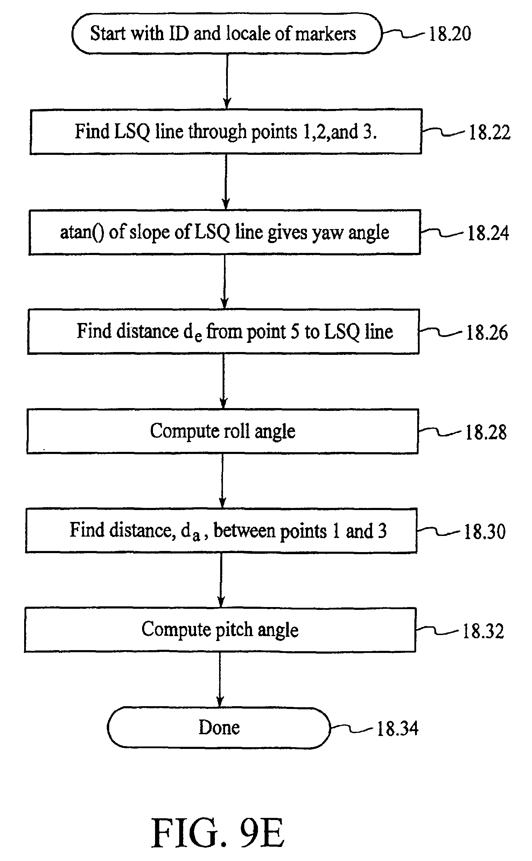

With reference also to Fig. 9E, an additional method for calculating the position and orientation of the energy delivery device 300, performed as part of step 18 of Fig. 9C, can be better understood. It is assumed prior to performing the steps of Fig. 9E that the individual position and orientation of markers 414A of catheter 400A have been determined by performing the steps of Fig. 9D for each individual marker 414A. The outcome of the steps of Fig.9E provides or describes the position and orientation of the distal marker in terms of yaw, roll, and pitch relative to a coordinate system defined by CPU 102, as is described in more detail below. The reference point, for example, can be provided by the fluoroscope planar view, preferably an RAO view, where the vertical axis of the RAO perspective is $x$, the horizontal axis of the RAO perspective is $y$, and the positive $z$ direction is defined as moving in-plane with respect to the $x$-$y$ plane, as shown if Fig. 4H.

In an initial step 18.20 the individual markers are provided with working identification numbers (IDs) or other descriptive information from which to distinguish one marker 414A from another marker 414A. For the purposes of discussion the markers are provided ID numbers ID1-ID5, ID1 given to the most proximal marker 414A and ID5 given to the most distal marker 414A, as shown in Fig. 4H.

It is important to note that the method of Fig. 9E minimally requires three markers 414A, however, the placement of additional markers 414A along the catheter 400A, along with corresponding analysis in accordance with other methods of the present invention, provide for a more accurate determination of position and orientation of the distal portion of the catheter 400A.

In a step 18.22 a best fit line using a least squares approach is determined through the more proximal markers 414A of the longitudinal shaft of the catheter 400A, represented here by markers 414A having IDs ID1-ID3. The best fit line shown as line 'L'.

In a step 18.24, the line L is compared with the $y$-$z$ planar surface, the angle formed therebetween defining a yaw orientation of the catheter 400A. Next, in a step 18.26 a line D perpendicular to the line L and passing through the distal marker 414A having ID5 is then generated, and the distance $d_e$ representing the length of line D with respect to the current perspective view is computed. With the distal portion 416 of catheter 400A having a predetermined shape, the maximum value of $d_e$, which is known or otherwise can be calculated during a calibration process, will be observed when the deflection plane of the catheter 400A is parallel to the plane of view. As the catheter 400A is rotated about the longitudinal axis of its main shaft, shown as line L, the distance $d_e$ measured will decrease. The roll angle can then be measured by the equation:

$$Roll = 90° - Cos^{-1}\left(\frac{d_e}{d_{e\,90°}}\right)$$

Where: $d_{e90}$ is the maximum value of $d_e$.

In a step 18.30, the distance $d_a$ between two or more markers 414A along line L is determined. As with the measurement $d_e$ above, since the orientation and placement of markers 414A along the longitudinal axis of the catheter 400A are known, the distance between these markers when the longitudinal axis is parallel to the perspective plane is also known. Pitch, defined as the angle formed by line L and the *x-y* planar surface, is then measured in a step 18.32 by computation of the equation:

$$Pitch = Cos^{-1}\left(\frac{d_a}{d_{a\,0°}}\right)$$

Where: $d_{a0}$ is the maximum value of $d_a$.

In a step 18.34 control is passed back to step 18 of Fig. 9C, the yaw, roll, and pitch representations being utilized by CPU 102 to more accurately define the position and orientation of catheter 400A. As shown in Fig. 4H, catheter 400A may include a bridge radio opaque member 414B between the two most proximal markers 414A, and described elsewhere herein, to help CPU 102 better determine in-plane and out-of-plane orientations of the distal portion 416. Additionally, it should be clear that the steps of Fig. 4H, while described in terms of catheter 400A, are applicable to the analysis of any energy delivery device comprising at least three radio opaque markers arranged at known orientation with respect to each other such as markers 414A having IDs ID1, ID2, and ID5. The markers may be of any suitable shape allowing for the calculations described above with respect to Fig. 9E.

With the yaw, roll, and pitch angles computed for the catheter 400A with reference to the origin of a three-dimensional coordinate system, CPU 102 can then merge this data with other data acquired in the current working image. The working image can then be combined with the live fluoro view and provided as one of the outputs of CPU 102. It should be understood that, while the actual measurements are made in terms of pixels of an image, since the resolution of the image is known, the measurements can be defined in terms of other units of length.

Additionally, if it is desired, the distance the energy delivery device or other item incorporating radio opaque material is from the X-ray source, not shown, can be calculated in an additional step (not shown). The equation that describes the relationship between an object's size and its imaged size in the fluoroscope system is:

$$u = x[D/(D-Z)]$$

Where:

$D$ is the source-to-detector distance;
$z$ is the object-to-detector distance;
$x$ is the object size; and
$u$ is the imaged size.

It is important to understand that relative position and orientation information regarding energy delivery devices, depictions of the heart, and other items utilized during a surgical procedure as described herein in accordance with the present invention, can be associated by way of additional analysis. For example, the planar perspectives, while being generally described with respect to RAO and LAO views, may also have other angular components, including cranial and caudal angles.

It will be understood that contemplating the use of delectable and shaped energy delivery devices having or comprising sections of increased or decreased radio opacity, when used in accordance with the present invention, will provide for enhanced visualization, recognition and control of the energy delivery device during the surgical procedure. Thus, when the tip of a catheter, for example, is rotated towards the septal or lateral walls while being viewed from an RAO perspective, it will be understood that the physical amount of material present between the X-ray source and the image detector of the fluoroscope will be increase and a fluoroscopic image thereof will appear increasingly opaque, i.e., the change in opacity can be correlated with the degree of rotation as well as the position of the tip and other portions.

With reference to Fig. 4I a representative RAO and LAO projection view of the distal end 710 of an energy delivery device 700, having a radio opaque marker 712 similar to marker 312, in the left ventricle with a set of radially asymmetrically spaced radio opaque markers 718 is shown being deflected toward the lateral surface of the heart. In similar fashion, Fig. 4J is a representative RAO and LAO view depicting the distal portion being deflected toward the septal surface of the heart. As described above, with the distal portion of the energy delivery device 700 oriented as shown in Figs. 4I and 4J, the distal tip marker 712 appears as a circle in the live fluoro. Note that a portion of the marker 712 in Fig. 4I is shown in dashed only to provide a clearer understanding that the distal portion of energy delivery device 700 is directed toward the lateral surface of the heart.

The markers 718 are thin stripe-type markers having centers located approximately 90° radially from the direction of tip deflection, the inner bend radius as defined above for example. Therefore, the deflection of the distal portion of energy delivery device 700 in-plane and out-of-plane with respect to the RAO view can be distinguished.

As depicted in the RAO view of Fig. 4I, it can be determined that the energy delivery device is shown deflected toward the lateral surface of the left ventricle since the markers 718 are viewed on the top surface of the energy delivery device depiction. In contrast, in the RAO view of Fig. 4J, the distal portion of the energy delivery device is deflected toward the septal surface since the markers 718 are viewed on the bottom surface of the energy delivery device depiction providing an indication that the energy delivery device has been rotated 180°.

Therefore, using methods of the present invention, CPU 102 can analyze the distal tip marker 712 along with the markers 718 of the RAO depictions of Figs. 4I and 4J, and from these views determine the position and orientation of the distal portion 716 of energy delivery device 700.

With reference back to Fig. 1, a fifth embodiment of an enhanced tracking system in accordance with the present invention, incorporating features described above pertaining to the general embodiment, can be more readily understood. The fifth embodiment of the present invention differs from the other embodiments in that the input instrument incorporates a localized location and orientation system utilizing magnetic sensors arranged on the distal portion of an energy delivery device. Such a system, by way of example, is disclosed in U.S. Patent 5,879,297 to Haynor et al. (hereinafter Haynor), entitled "System and Method to Determine the Location and Orientation of an Indwelling Medical Device."

The system of Haynor comprises a housing and, using magnetic field analysis, can provide an output descriptive of the location of the energy delivery device with respect to the housing. This output would then be provided to the CPU 102 of the present invention and, along with other data acquired, merged with one or more working image and combined with other information to generate one or more output, as discussed in more detail above. The output provided to CPU 102 could comprise, for example, a video signal providing depicting the current distal tip position and relative treatment site locations. Alternatively, the provided output could comprise binary data descriptive of the relative positions of the distal tip position of the energy delivery device and treatment site locations. In both cases, however, the output would be referenced to a three-dimensional origin relative to the housing of the Haynor device.

CPU 102, in order to generate one or more output, must be able to correlate the data received from the Haynor system with other data acquired and integrated into the current working images. This may be achieved by placing additional magnetic sensors at know relationships with respect to other objects or sensors from which CPU 102 acquires data. For example, magnetic sensors could be arranged at the center of the motion markers described above. Additionally, the single radio opaque marker on the distal tip of the energy delivery device at a known relationship with respect to the magnetic sensors placed thereon can provide a suitable data reference. Both of the above examples provide CPU 102 a reference point from which output data received from the Haynor system can be correlated to other data obtained data.

At this point it should be appreciated that with increased computing power there will exist a corresponding increase in complexity with respect to the output depictions of the present invention. For example, the three dimensional output depiction described above could be an actual generated depiction of the heart itself. The heart being able to be rotated and otherwise manipulated to depict the position and orientation of an energy delivery device therein.

While the principles of the invention have been made clear in illustrative embodiments, there will be immediately obvious to those skilled in the art many modifications of structure, arrangement, proportions, the elements, materials, and components used in the practice of the invention, and otherwise, which are particularly adapted to specific environments and operative requirements without departing from those principles. The appended claims are intended to cover and embrace any and all such modifications, with the limits only of the true purview, spirit and scope of the invention.

**EP 1 057 455 A2**

**Claims**

1. A method for tracking a surgical device in a live fluoro view during a surgical procedure comprising the steps of:

   a) Generating at least one working image from a live fluoro video signal corresponding to a first of at least one perspective view;
   b) Acquiring body systems data, the body system data being registered with the at least one working image;
   c) Detecting at least one event from which additional data related to the surgical procedure is acquired, the additional data being merged into the at least one working image;
   d) Combining the generated at least one working image with the live fluoro video signal and forming a first of a plurality of outputs, the first output being a video signal; and
   e) Providing the first output to a display system wherein the first output can be depicted on at least one video display.

2. The method of claim 1 wherein the step of combining the generated at least one working image with the live fluoro video signal comprises the step of storing the at least one working image, wherein the stored data provides a histology of the surgical procedure.

3. The method of claim 1 wherein the step of generating at least one working image from a live fluoro video signal comprises the step of digitizing a video frame as part of the live fluoro video signal representative of the first of the at least one perspective view.

4. The step of claim 2 wherein a first of the at least one event is a motion marker detection event, data obtained in the step of detecting at least one event comprises position and orientation information with respect to at least one motion marker, the at least one working image being oriented with the live fluoro video signal.

5. The step of claim 4 wherein a second of the at least one event is a ventriculogram event, data obtained in the step of detecting at least one event further comprises data representative of at least one outline view corresponding to the first of the at least one perspective view, the at least one outline view being stored.

6. The step of claim 5 wherein a third of the at least one event is an surgical device detection event, the step of detecting at least one event further comprising the step of acquiring the position of the distal portion of the surgical device, data obtained in the step of detecting at least one event further comprises position data of the distal portion of the surgical device.

7. The step of claim 6 wherein a fourth of the at least one event is a treatment event, data obtained in the step of detecting at least one event further comprises position data of the distal portion of the surgical device during the application of a desired therapy.

8. The method of claim 5 further comprising a first and a second of the at least one outline views, the first of at least one outline view being a right anterior oblique-30° view and a second of at least one outline view being a left anterior oblique-60° view.

9. The method of claim 8 wherein the step of acquiring the position of the distal portion of the surgical device further comprises the steps of:

   a) Defining a sequence of images comprising at least two working images depicting a specific perspective view of the live fluoro;
   b) Obtaining a coordinate representative of the position of the distal portion of the surgical device within the current working image;
   c) Extracting a subimage from the current working image, the subimage being a predetermined distance from the coordinate;
   d) Detecting the distal portion of the surgical device within the subimage, wherein a new coordinate defining a central portion of the distal portion of the surgical device is defined with respect to the current working image;
   e) Estimating the position of the distal portion of the surgical device in a next working image as part of the sequence of images; and
   f) Repeating steps c through e until all the images of the sequence have been considered, the next working image becoming the current working image.

**10.** The method of claim 9 wherein the step of detecting the distal portion of the surgical device with the subimage comprises an initial step of filtering the subimage.

**11.** The method of claim 10 wherein the step of estimating the position of the distal portion of the surgical device comprises the step of calculating velocity vectors representative of the distal portion of the surgical device.

**12.** The method of claim 11 wherein the step of detecting the distal portion of the surgical device within the subimage further comprises the step of comparing the subimage with at least one known depiction of the distal portion of the surgical device.

**13.** The method of claim 12 wherein the step of detecting the distal portion of the surgical device within the subimage further comprises the final step of storing the coordinate.

**14.** The method of claim 13 wherein the step of acquiring position data of the distal portion of the surgical device further comprises the steps of:

a) Detecting the orientation of the distal portion of the surgical device; and
b) Translating the orientation into out-of-plane or in-plane coordinates representative of the distal portion of the surgical device.

**15.** The method of claim 14 wherein the step of comparing the subimage with at least one known depiction of the distal portion of the surgical device comprises the steps of:

a) Defining at least two depictions of the distal portion of the surgical device, a current depiction defined by a first of the at least two depictions;
b) Initializing an output variable corresponding to a selected depiction to zero;
c) Applying a mean-square-difference filter to the subimage and the current depiction;
d) Determining the orientation of the distal portion of the surgical device; and
e) Repeating steps b through c until all depictions have been considered, the output value being descriptive of the position of the distal portion of the surgical device.

**16.** The method of claim 15 wherein the mean-square-difference filter is defined as:

$$M(a,b) = \frac{1}{KL} \sum_{k=-K/2}^{K/2} \sum_{l=-L/2}^{L/2} [f_m(a+k,b+l) - h(k,l)]^2$$

Where:

$h(k,l)$ = Value of the current depiction at pixel location $(k,l)$;
L = Length of the depiction in pixels; and
K = Width of the depiction in pixels.

**17.** The method of claim 15 wherein the step of determining the orientation of the distal portion of the surgical device comprises the steps of:

a) Calculating a reciprocal value of the result obtained from the application of the mean-square-filter;
b) Calculating a degree of similarity value descriptive of the degree of similarity between the subimage and the current depiction by applying a two-dimensional Gaussian function to the reciprocal value; and
c) Setting the output variable to the degree of similarity value if the degree of similarity value is larger than the output variable.

**18.** The method of claim 17 wherein the step of determining the orientation of the distal portion of the surgical device comprises the steps of:

a) Calculating an equation of a first line, using a mathematical method, the line passing through at least two radio opaque markers which are positioned on the longitudinal axis of the surgical device, the first line, when compared to a first planar surface defined by two axes of a coordinate system, defining a first angle;

b) Calculating an equation of a second line, using a mathematical method, the second line being tangential to the first line and passing through a radio opaque marker on the distal tip of the surgical device, the length of the second line being representative of an angle of rotation about the longitudinal axis of the surgical device;

c) Calculating a distance between the at least two radio opaque markers which are positioned on the longitudinal axis of the surgical device, the distance being representative of a second angle formed between the longitudinal axis of the surgical device and a second planar surface defined by two axes of the coordinate system, the second planar surface being orthogonal to the first planar surface.

**19.** An enhanced surgical device tracking system comprising:

a central processing unit;

at least one input instrument, comprising at least one sensor in combination with a surgical device, the at least one input instrument operably interfaced with the central processing unit and providing the central processing unit raw data descriptive of the relative position and orientation of the surgical device;

at least one input device operably interfaced with the central processing unit, the at least one input device providing the central processing unit additional data descriptive of a surgical procedure being performed, the central processing unit being able to generate at least one working image corresponding to a specific perspective view from the raw data and additional data acquired; and

a video display system operably interfaced with the central processing unit and adapted to accept and display at least one output signal from the central processing unit, a first of the at least one output signal being a video signal comprising the at least one working image with a live fluoro video signal of the same specific perspective view.

**20.** A method for tracking a surgical device in a live fluoro view during a percutaneous transluminal myocardial revascularization procedure comprising the steps of:

a) Acquiring data descriptive of the position and orientation of the surgical device from one or more input instruments and one or more input devices;

b) Processing the acquired data; and

c) Generating at least one output from the processed data.

**21.** A computer program product for tracking a surgical device during a surgical procedure, the computer program product comprising:

a computer usable medium having computer readable program code embodied in the medium for use in a computer in a surgical device tracking system, the surgical device tracking system including a central processing unit, at least one input instrument comprising at least one sensor in combination with a surgical device operably interfaced with and providing the central processing unit raw data descriptive of the relative position and orientation of the surgical device during the surgical procedure being performed, at least one input device operably interfaced with and providing the central processing unit additional data descriptive of the surgical procedure being performed, and a video display system operably interfaced with the central processing unit and adapted to accept and display at least one output signal from the central processing unit, the program code comprising:

first code that causes the central processing unit to receive input from the at least one input instrument;

second code that causes the central processing unit to receive input from the at least one input device;

third code that causes the central processing unit to generate at least one working image from the data received from the at least one input instrument and the at least one input device;

fourth code that causes the central processing unit to combine the generated working image with a live fluoro video signal, forming a combined signal; and

fifth code that causes the central processing unit to deliver the combined signal to the video display system for displaying the combined signal as a first of the at least one output signal from the central processing unit.

FIG. 1

EP 1 057 455 A2

FIG. 2C

FIG. 2A

FIG. 2B

FIG. 3A

FIG. 3B

FIG. 4A

EP 1 057 455 A2

FIG. 4B

FIG. 4C

FIG. 4D

EP 1 057 455 A2

402    414    416    414    412

410

400    FIG. 4E

402    416
414    414    412    410

400    FIG. 4F

414

402    416
414B    414    C    D    412    410
414    A    B    414

400    FIG. 4G

410

412    D
I05

I04    414A

I01    414B    I02    I03    L

414A    414A    400A    d_e
d_a

FIG. 4H

FIG. 4I

FIG. 4J

FIG. 5A

FIG. 5B

EP 1 057 455 A2

FIG. 6

**FIG. 7A**

**FIG. 7B**

**FIG. 7C**

**FIG. 7D**

FIG. 8A

FIG. 8B

FIG. 8C

Digitize Frame from Flouro —150

Register Frame with Body Systems —152

Motion Marker Detection in Progress ? —160

Acquire Motion Marker Location —162

Orient Working Images —164

V-gram in Progress ? —170

Detect Outline Views of Left Ventricle —172

Store Outline Images —174

Catheter Detection in Progress ? —180

Acquire Catheter Location —182

Update Working Images —188

Treatment in Progress ? —200

Acquire Treatment Location —202

Update Working Images —208

Combine Working Images with Live Flouro —210

Provide Selected Outputs —220

FIG. 9A

From Step 170 or 174

182          184          186        188

Catheter Detection in Progress ?    180

Acquire Catheter Location

Detect Sensor Orientations

Translate Sensor Orientations into In-plane or Out-of-plane Coordinates

Update Working Images

To Step 190

FIG. 9B

EP 1 057 455 A2

| Obtain initial estimate of the tip position $(x_x, y_y)$ | 10 |

| $V_x = 0. \quad V_y = 0. \quad i = 0.$ | 12 |

| Extract subimage $f$ of $I$ around estimate $(x_x, y_y)$ | 14 |

| Filter the subimage $f$: $f_m = f \cdot$ medianfilt $(f)$; | 16 |

| Locate Distal Portion Marker | 18 |

| Update $x(i)$ and $y(i)$ with new position. | 20 |

| If $i > 0$ $V_x = x(i) \cdot x(i-1)$, $V_y = y(i) \cdot y(i-1)$. | 22 |

| Estimate position in next frame $x_x = x(i) + V_x$ $y_x = y(i) + V_y$ | 24 |

| $i = i + 1$ | 26 |

N — At end of Sequence ? — 28

Y

# FIG. 9C

$N_k$ = # of kernels to match — 18.0

$j = 0$ — 18.2

$M = MSD[f_m, h(j)]$ — 18.4

$Z = I/M$ — 18.6

$Z_G = Z \times G_{2d}$ — 18.8

Save the maximum of $Z_G$ and the location of this maximum. — 18.10

$j = j + 1$ — 18.12

18.14

$j < N_k$

Y

N

The tip location is the location of the maximum of all maxima. — 18.16

## FIG. 9D

Return the tip location — 18.18

Start with ID and locale of markers — 18.20

Find LSQ line through points 1,2,and 3. — 18.22

atan() of slope of LSQ line gives yaw angle — 18.24

Find distance $d_e$ from point 5 to LSQ line — 18.26

Compute roll angle — 18.28

Find distance, $d_a$, between points 1 and 3 — 18.30

Compute pitch angle — 18.32

Done — 18.34

# FIG. 9E

FIG. 10A

FIG. 10B

FIG. 10C

FIG. 10D